(19) Europäisches Patentamt
European Patent Office
Office européen des brevets

(11) Veröffentlichungsnummer: **0 360 369 B1**

(12) # EUROPÄISCHE PATENTSCHRIFT

(45) Veröffentlichungstag der Patentschrift: **03.05.95**

(51) Int. Cl.[6]: **C07J 15/00**, C07J 41/00, C07J 53/00

(21) Anmeldenummer: **89250040.6**

(22) Anmeldetag: **20.09.89**

Die Akte enthält technische Angaben, die nach dem Eingang der Anmeldung eingereicht wurden und die nicht in dieser Patentschrift enthalten sind.

(54) **11Beta-phenyl-14betaH-steroide.**

(30) Priorität: **20.09.88 DE 3832303**

(43) Veröffentlichungstag der Anmeldung:
**28.03.90 Patentblatt 90/13**

(45) Bekanntmachung des Hinweises auf die Patenterteilung:
**03.05.95 Patentblatt 95/18**

(84) Benannte Vertragsstaaten:
**AT BE CH DE ES FR GB GR IT LI LU NL SE**

(56) Entgegenhaltungen:
**EP-A- 0 057 115**
**EP-A- 0 116 974**
**EP-A- 0 277 676**
**EP-A- 0 283 428**
**ZA-A- 8 801 962**

(73) Patentinhaber: **SCHERING AKTIENGESELL-SCHAFT**

**D-13342 Berlin (DE)**

(72) Erfinder: **Scholz, Stefan, Dr.**
**Lützenstrasse 9**
**D-1000 Berlin 31 (DE)**
Erfinder: **Neef, Günter, Dr.**
**Markgraf-Albrecht-Strasse 4**
**D-1000 Berlin 15 (DE)**
Erfinder: **Ottow, Eckhard, Dr.**
**Moltkestrasse 48**
**D-1000 Berlin 45 (DE)**
Erfinder: **Elger, Walter, Dr.**
**Schorlemer Allee 12B**
**D-1000 Berlin 33 (DE)**
Erfinder: **Beier, Sybille, Dr.**
**Uhlandstrasse 121**
**D-1000 Berlin 31 (DE)**
Erfinder: **Chwalisz, Krzysztof, Dr.**
**Luzerner Strasse 1d**
**D-1000 Berlin 45 (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 11β-Phenyl-14βH-steroide der allgemeinen Formel I

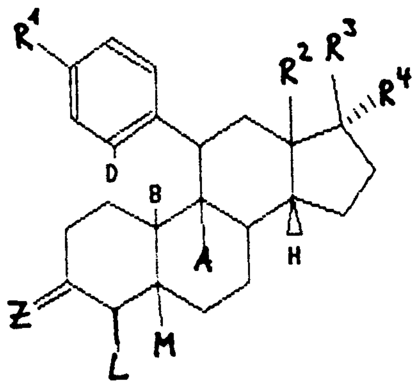

(I),

worin

Z für eine Sauerstoffatom oder die Hydroxyiminogruppierung N~OH sowie L und M entweder gemeinsam für eine zweite Bindung oder L für ein Wasserstoffatom und M für eine α-ständige Hydroxygruppe stehen und entweder A und B zusammen eine zweite Bindung und D ein Wasserstoffatom bedeuten, wobei

$R^1$ entweder

a) für einen Heteroarylrest der Formel Ia

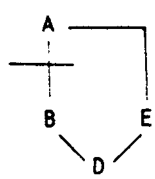

(Ia),

wobei A = N, O oder S und
B-D-E die Elementenfolge
C-C-C, N-C-C oder C-N-C
bedeuten oder

b) für einen Heteroarylrest der Formel Ib

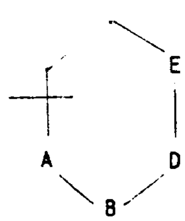

(Ib),

wobei A = N und
B-D-E die Elementenfolge
C-C-C, N-C-C, C-N-C oder C-C-N
bedeuten oder

c) für einen Cycloalkyl-, Cycloalkenyl- oder Arylrest Ic stehen

oder aber A ein Wasserstoffatom und B und D zusammen eine das C-10-Atom des Steroidgerüstes und das ortho-Kohlenstoffatom des 11ß-Phenylringes überbrückende Methylengruppe bedeuten, wobei dann

Z zusätzlich für 2 Wasserstoffatome steht und

$R^1$ entweder die vorstehend in diesem Anspruch unter a), b) oder c) genannte Bedeutung hat oder

d) für einen geradkettigen, verzweigten, gesättigten oder ungesättigten Kohlenwasserstoffrest mit bis zu 10 Kohlenstoffatomen oder

e) für

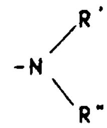

mit R′ und R″ in der Bedeutung von Wasserstoff oder Alkyl mit 1 bis 4 Kohlenstoffatomen oder R′ und R″ unter Einschluß von N in der Bedeutung eines gesättigten 5- oder 6gliedrigen Ringes, wobei im Ring außer N noch ein weiteres Heteroatom wie O, N, S enthalten sein kann, sowie die entsprechenden tertiären N-Oxide und die Säureadditionssalze oder

f) für OR‴ mit R‴ = H, $C_1$-$C_8$-Alkyl oder

g) für $SR^{IV}$ mit $R^{IV}$ in der Bedeutung von R‴ oder

h) für einen geradkettigen, verzweigten, gesättigten oder ungesättigten Kohlenwasserstoffrest mit bis zu 10 Kohlenstoffatomen, der durch eine oder mehrere Oxo-, Hydroxyimino-, $C_1$-$C_{10}$-Acyloxy- oder $OR^V$-Gruppe(n) mit $R^V$ in der Bedeutung eines Wasserstoffatoms oder eines $C_1$-$C_8$-Alkylrestes substituiert ist,

steht und wobei gegebenenfalls der Heteroarylrest der Formel Ia durch einen oder mehrere Halogenreste und/oder einen oder mehrere Alkylreste mit 1 bis 3 Kohlenstoffatomen und der Cycloalkyl-, Cycloalkenyl- oder Arylrest Ic durch eine oder mehrere Halogen-, gegebenenfalls geschützte Hydroxy-, Alkoxy-, gegebenenfalls in Form des Sulfoxids oder Sulfons und/oder des N-Oxids oxidierte Alkylthio- und/oder Dialkylaminoreste substituiert sind,

$R^2$ für einen Methyl- oder Ethylrest,

$R^3/R^4$ für $-(CH_2)_o-C{\equiv}CY/OR_5$ $-CH=CH-(CH_2)_kCH_2R_7/-OR_5$

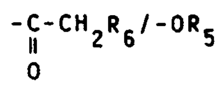

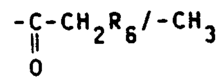

$-(CH_2)_oCH_2R_7/-OR_5$

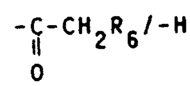

sowie, wenn A ein Wasserstoffatom und B und D zusammen eine, das C-10 Atom des Steroidgerüstes und das ortho-C-Atom des 11β Phenylringes überbrückende Methylengruppe bedeuten,

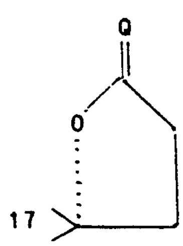

oder

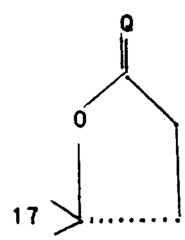

mit $R_5$ in der Bedeutung eines Wasserstoffatoms oder Acylrestes mit 1 bis 4 Kohlenstoffatomen und Y in der Bedeutung eines Wasserstoff-, Chlor-, Jod- oder Bromatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,

$R_6$ für ein Wasserstoffatom, eine Hydroxygruppe, eine Alkyl-, O-Alkyl-oder O-Acylgruppe mit jeweils 1 bis 4 Kohlenstoffatomen,

O für die Ziffern 0, 1, 2 oder 3,

$R_7$ für einen Hydroxy- oder Cyanidrest, eine O-Alkyl- oder O-Acylgruppe mit jeweils 1 bis 4

Kohlenstoffatomen,

k für die Ziffern 0, 1 oder 2 und

Q für ein Sauerstoffatom oder zwei Wasserstoffatome

stehen.

Als besonderes Merkmal weisen die Verbindungen vorliegender Erfindung in Abweichung von den natürlich vorkommenden Steroiden am 14-Kohlenstoffatom eine β-ständiges Wasserstoffatom auf. Solche 14β-H-Steroide sind bereits kürzlich in begrenztem Umfang aus der Europäischen Patentanmeldung 0 277 676 bekannt geworden. Diese bekannten Verbindungen mit $\Delta^{4,9}$-3-Keto-Steroidgrundgerüst weisen in 11-Position einen p-substituierten (Hetero)Arylring auf. Als p-Substituenten sind beschrieben: wahlweise gesättigte oder ungesättigte, geradkettige oder verzweigte Kohlenwasserstoffreste mit 1 - 10 Kohlenstoffatomen, wobei der Kohlenwasserstoffrest zusätzlich noch eine Hydroxyimino-, Oxo- und/oder Hydroxygruppe tragen kann

oder eine

Aminogruppe -NXY, mit X und Y = H oder $C_1$-$C_4$-Alkyl, wobei wenn X und Y = Alkyl, diese auch gemeinsam mit N einen heterocyclischen 3- bis 7-Ring bilden können.

Das gemäß EP-A 0 277 676 am 11β-Phenylring erhältiche Substitutionsmuster ist relativ begrenzt. Die Suche nach weiteren 14β-H-Steroiden, die am 11β-Phenylring über den Rahmen der EP-A 0 277 676 hinausgehende Substituenten aufweisen, scheint deshalb dringend geboten, um zu Verbindungen zu gelangen, die hinsichtlich ihrer zum Beispiel antigestagenen Wirkung denen der EP-A 0 277 676 überlegen sind.

Die neuen Verbindungen der allgemeinen Formel I sowie deren pharmazeutisch verträgliche Additionssalze mit Säuren sind wertvolle Pharmaka. So verfügen sie über eine starke Affinität zum Gestagenrezeptor und besitzen antigestagene Eigenschaften.

Die antiglucocorticoide Wirksamkeit ist nur noch schwach ausgeprägt oder gar nicht mehr vorhanden (im Vergleich zu dem aus der EP-A 0 057 115 bekannten 11β-(4-Dimethylaminophenyl)-17β-hydroxy-17α-(propin-1-yl)-4,9(10)-estradien-3-on (RU 486)).

Im Gestagenrezeptor-Bindungstest wird die Affinität der erfindungsgemäßen Verbindungen zum Gegenrezeptor untersucht. Gemessen wird dabei die Verdrängung des Agonisten durch den Antagonisten.

Man verwendet Cytosol aus Kaninchenuterushomogenat, das das Rezeptormolekül - ein Protein - enthält. Dieses bindet mit hoher Affinität und geringer Kapazität Progesteron. Wenn diese Rezeptoren mit $^3$H-Progesteron in Gegenwart der zu prüfenden, unmarkierten Substanz beladen werden, so hängt es von der Konzentration und von der Bindungsaffinität der zu untersuchenden Verbindung ab. wie stark $^3$H-Progesteron vom Rezeptor verdrängt wird. Nach Trennung des Rezeptor-gebundenen Progesterons vom nichtgebundenen kann man die Bindung in Prozent ermitteln und diesen Wert gegen den Logarithmus der molaren Konzentration der Prüfsubstanz auftragen. Man erhält charakteristische dosisabhängige Verdrängungskurven und kann nun die Konzentration der Prüfsubstanz ermitteln, die erforderlich ist, um die Referenzsubstanz vollständig vom Rezeptor zu verdrängen. Der Kompetitionsfaktor K als Maß für die Bindungsstärke ist definiert als das Verhältnis der Konzentration der Prüfsubstanz zur Konzentration der Referenzsubstanz (Progesteron), bei der beide Verbindungen eine gleich große Verdrängung von $^3$H-Progesteron vom Progesteron-Rezeptorkomplex zeigen.

TABELLE 1

| Gestagen-Rezeptor-Bindungstest | |
|---|---|
| Verbindung | Kaninchenuterus K (gestagen) |
| A | 11,2 |
| B | 4,2 |
| C | 15 |
| D | 7 |
| E | 13 |
| F | 5,5 |

A = 11β-(4-Acetylphenyl)-17α-hydroxy-17-( prop-1-inyl)-14β-estra-4,9-dien-3-on,
B = 11β-(4-Acetylphenyl)-17α-hydroxy-17-(prop-2-inyl)-14β-estra-4,9-dien-3-on,
C = 17-Hydroxy-17β-(3-hydroxypropyl)-11β,19-(4-methoxy-o-phenylen)-14β-androst-4-en-3--on,
D = 17-Hydroxy-17β-(3-hydroxypropyl)-11β,19-[4-(3-pyridyl)-o-phenylen]-14β-androst-4-en--3-on,
E = 17-Hydroxy-17β-(3-hydroxypropyl)-11β,19-(4-vinyl-o-phenylen)-14β-androst-4-en-3-on,
F = 11β-(4-Dimethylaminophenyl)-17α-hydroxy-17-(3-hydroxypropyl)-14β-estra-4,9-dien-3--on (Vergleichsverbindung, EP-A 0 277 676).

Wirkstoffe dieser Art mit antigestagener Aktivität können in erster Linie für die Behandlung von hormonabhängigen Tumoren, welche Progesteronrezeptoren in ihrem Gewebe aufweisen, eingesetzt werden. Bevorzugte diesbezügliche Indikation ist die Behandlung des Mamma-Carcinoms.
Sie sind aber auch zur Auslösung von Aborten geeignet, da sie das zur Aufrechterhaltung der Schwangerschaft erforderliche Progesteron vom Rezeptor verdrängen. Sie können zur postcoitalen Fertilitätskontrolle, Menstruationsauslösung und zur Geburtseinleitung dienen.
Schließlich können sie auch bei der Behandlung hormoneller Unregelmäßigkeiten Verwendung finden.

Die antiproliferative Potenz der Progesteronantagonisten auf die Brustdrüse wird in einem Bioassay ermittelt. Zu diesem Zweck werden ovariektomierte Ratten 3 Tage lang mit Estron (10 μg) und Progesteron (3 mg) substituiert und eine weitere Gruppe gleichzeitig mit nachfolgenden Progesteronantagonisten behandelt (1 mg/Tier/Tag):

G: 11β-(4-Acetylphenyl)-17β-hydroxy-17α-(1-propinyl)-4,9-estradien-3-on,
H: 11β-[4-(3-Pyridyl)phenyl]-17α-hydroxy-17-(3-hydroxypropyl)-14β-estra-4,9-dien-3-on,
I: 11β-(4-Dimethylaminophenyl)-4,5-dihydro-spiro-[14β-estra-4,9-dien-17α,2′(3′H)-furan]-3-on (EP-A-0 277 676).

Die gesamte inguinale Mamma wird zur Durchführung der "whole mount"-Technik (Lyons and Johnson, 1952) herauspräpariert. Von sechs Tieren wird die Anzahl der tubulo-alveolären Drüsenendstücke mittels 40-facher Vergrößerung bestimmt.

Die antiproliferative Potenz wird in Prozent der Hemmung der tubulo-alveolären Endstücke entsprechend der folgenden Formel ausgedrückt:

(Estron (E) + Progesteron (P)) - (E + P + G) = 100%

behandelte Tiere

(E + P) - (Testverbindung H oder I) = X%
100% - X% = % Hemmung

G: 100% Hemmung
H: 92% Hemmung

I: 13% Hemmung

Die erfindungsgemäßen Verbindungen der allgemeinen Formel I sowie deren pharmazeutisch verträgliche Additionssalze mit Säuren weisen lediglich eine geringe oder gar keine antiglucocorticoide Aktivität auf verglichen mit RU 486 (s.o.), welches als Standard anzusehen ist (7th Int. Congress of Endocrinology, July 1-7. 1984, Quebec City, Canada; Excerpta Medica, Amsterdam-Oxford-Princeton).

Zur Kennzeichnung der antiglucocorticoiden Wirkung wurde der Einfluß der erfindungsgemäßen Substanzen auf die Tyrosin-Aminotransferase bestimmt. Das Test-System basiert auf einer Messung der Aktivität des Leberenzyms Tyrosinaminotransferase (TAT) in Kulturen von RHC (Rat Hepatoma Cells) Zellen. Das Enzym katalysiert den ersten Schritt in der Verstoffwechselung von Tyrosin und ist sowohl in der Leber als auch in Hepatomzellen durch Glucocorticoide induzierbar. Die Aktivität ist in Rohextrakten leicht meßbar (Granner und Tomkins, (1970) Meth. Enzymol. 15, 633). Das Enzym überführt die Aminogruppe von Tyrosin auf 2-Oxoglutarsaure. Dabei entstehen Glutaminsäure und p-Hydroxyphenylpyruvat. In alkalischer Lösung wird aus p-Hydroxyphenylpyruvat der stabilere p-Hydroxybenzaldehyd gebildet, dessen Absorption bei 331 nm gemessen wird. Die TAT-Aktivität in RHC-Zellen zeigt eine dosisabhängige Induktion mit Cortisol (max. Akt. bei $10^{-6}$ M) oder Dexamethason (max. Akt. bei $10^{-7}$ M). Die Aktivität läßt sich um den Faktor 4-6 über den Basalwert stimulieren. Gleichzeitige Behandlung mit Corticoid und Antiglucocorticoid führt zu einer Abnahme der TAT-Aktivität.

Die Ergebnisse des oben beschriebenen Tests für die Verbindungen A bis F sind in Tabelle 2 zusammengefaßt.

TABELLE 2

| Antiglucocorticoide Wirksamkeit der Verbindungen A bis G; die Wirksamkeit ist in % der Wirksamkeit von RU 486 (Wirksamkeit 100%) angegeben | |
|---|---|
| Verbindung | Antiglucocorticoide Wirksamkeit (in % von RU 486) |
| A | 1 |
| B | 1 |
| C | 0 |
| D | 0 |
| E | 1 |
| F | 2 |

Die Erfindung betrifft somit auch Arzneimittel auf der Basis der pharmazeutisch verträglichen, d.h. in den verwendeten Dosen nicht toxischen Verbindungen der allgemeinen Formel I sowie deren Additionssalze mit pharmazeutisch verträglichen Säuren, gegebenenfalls zusammen mit den üblichen Hilfs- und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens$^{(R)}$ oder Myrj$^{(R)}$, Magnesiumstearat, wäßrigen oder nichtwäßrigen Trägern, Paraffinderivaten, Netz-, Dispergier-, Emulgier-, Konservierungsmitteln und Aromastoffen zur Geschmackskorrektur (z.B. ätherischen Öle) gemischt werden.

Die Erfindung betrifft somit auch pharmazeutische Zusammensetzungen, die als Wirkstoff zumindest eine erfindungsgemäße Verbindung oder eines ihrer pharmazeutisch verträglichen Additionssalze mit Säuren enthalten.

Als Additionssalze der erfindungsgemäßen Produkte mit Säuren sind insbesondere die Hydrochloride und die Methansulfonate zu nennen.

Eine Dosiseinheit enthält etwa 1-100 mg Wirkstoff(e).

Die Dosierung der erfindungsgemäßen Verbindungen liegt beim Menschen bei etwa 1-1000 mg pro Tag.

Von den gemäß Formel Ia möglichen Heteroarylresten sind der 3-Thienyl-, 3-Furyl- und 3-Pyrrolrest bevorzugt.

Als Heteroarylreste der Formel Ib kommen erfindungsgemäß insbesondere der 3- oder 4-Pyridyl-, der 5-Pyrimidin-, 4-Pyridazin- oder Pyrazinrest infrage.

Als Cycloalkyl-, Cycloalkenyl- oder Arylrest Ic sind der Cyclohexyl-, der Cyclohex-1-enyl-, Cyclohex-2-enyl-, Cyclohex-3-enyl- sowie der Phenylrest besonders hervorzuheben.

Der gemäß Formel I unter d) bzw. h) genannte Kohlenwasserstoffrest soll im ungesättigten Fall vorzugsweise bis zu 3 Doppelbindungen aufweisen.

Als Halogensubstituenten, die am Heteroarylrest der Formel Ia möglich sind, sind insbesondere ein Chlor- oder Bromatom zu nennen.

Ist der Heteroarylrest der Formel Ia alkylsubstituiert, so ist die Monosubstitution bevorzugt.

Der Cycloalkyl-, Cycloalkenyl- oder Arylrest Ic kann durch ein oder zwei Chlor- und/oder Bromatom(e) substituiert sein. Die genannten Reste können auch durch ein oder zwei, gegebenenfalls geschützte Hydroxy-und/oder Alkoxyreste mit 1 bis 8 Kohlenstoffatomen substituiert sein.

Die in

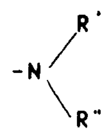

vorkommenden Alkylgruppen mit 1 - 4 Kohlenstoffatomen sind bevorzugt Methyl, Ethyl und/oder Propyl. Bilden R′ und R″ gemeinsam unter Einschluß des Stickstoffatoms einen heterocyclischen Fünf- oder Sechsring, der außer N- und C-Atome auch noch zusätzlich ein O- oder S-Atom enthalten kann, so seien hier beispielsweise der Pyrrol-, Pyrrolidin-, Piperidin-, Piperazin-, Morpholin-, Oxa- und Thiazolidin-sowie Thiadiazolidin-Ring genannt.

Als $R^2$-Substituent ist die Methylgruppe erfindungsgemäß bevorzugt.

Aus den erfindungsgemäß für $R^3/R^4$ möglichen Kombinationen ist keine besonders hervorzuheben, jedoch sind für die in den in $R^5$, Y, $R^6$ und $R^7$ vorkommenden Alkyl- und/oder Acylgruppen die Methyl-, Ethyl-, Propyl-bzw. Formyl-, Acetyl-, Propionyl- sowie Butyrylgruppe bevorzugt.

Bezüglich der Substituenten A, B, D sind zwei erfindungsgemäß mögliche Fälle zu unterscheiden:

1) A und B bilden gemeinsam eine zweite Bindung zwischen Kohlenstoffatom 9 und 10, und D steht für ein Wasserstoffatom. Man gelangt zu den $\Delta^{4,9}$-3-Keto-11β-phenyl-14β-H-steroiden der allgemeinen Formel I′

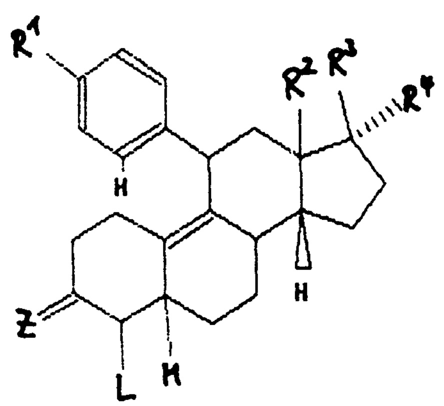

(I′);

$R^1$ steht hierin wie bereits angegeben für einen fünf- oder sechsgliedrigen Heteroarylrest der Formel Ia bzw. Ib oder für einen Cycloalkyl-, Cycloalkenyl- oder Arylrest Ic.

2) A steht für ein Wasserstoffatom und B und D bilden gemeinsam eine das C-10-Atom und das O-Kohlenstoffatom des 11β-Phenylringes überbrückende Methylengruppe. Man gelangt zu den $\Delta^4$-3-Keto-19,11β-o-phenylen-14β-H-steroiden der allgemeinen Formel I″

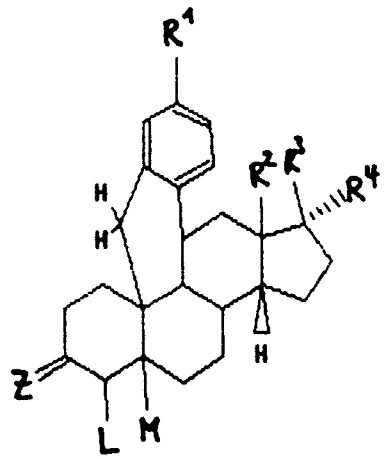

(I");

Zusätzlich zu den bereits unter Formel I′ für $R^1$ angegeben Substituenten steht $R^1$ auch noch für die in Formel I under d) bis h) beschriebenen Substituenten.

Schließlich stehen sowohl in Formel I′ als auch in I″ L und M für eine zweite Bindung zwischen dem C-4- und C-5-Atom oder L für ein H-Atom und M für eine α-ständige Hydroxygruppe.

Die neuen Verbindungen der allgemeinen Formel I werden erfindungsgemäß hergestellt, indem eine Verbindung der allgemeinen Formel II

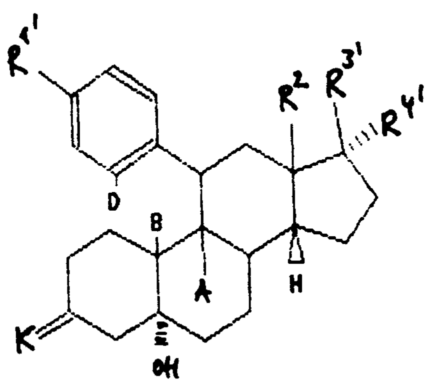

(II),

worin A, B, D und $R^2$ die in Formel I angegebene Bedeutung haben,

K eine in Form des Ketals oder Thioketals blockierte Ketogruppe bedeutet,

$R^{1'}$ entweder die bei $R^1$ unter a) bis g) angegebene Bedeutung hat, oder

einen geradkettigen oder verzweigten, gesättigten oder ungesättigten Alkylrest mit 1 bis 10 Kohlenstoffatomen, der die Gruppierung

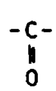

in der geschützten Form

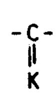

mit K in oben angegebener Bedeutung oder K in der Bedeutung eines Wasserstoffatoms und einer gegebenenfalls geschützten Hydroxygruppe enthält, sowie

$R^{3'}$ und $R^{4'}$

die gleiche Bedeutung haben wie $R^3$ und $R^4$ in Formel I, wobei vorhandene Hydroxy- und/oder Acyl-

und/oder terminale Alkingruppen gegebenenfalls geschützt sind,
der Einwirkung eines Dehydratationsmittels, das auch zur Freisetzung der 3-Oxogruppe und in $R^{1'}$ vorhandener geschützter Ketogruppen befähigt ist, gegebenenfalls unter Ausbildung der 4(5)-Doppelbindung unterwirft, das so erhaltene Produkt gegebenenfalls von weiteren Schutzgruppen befreit und gewünschtenfalls mit Hydroxylamin-Hydrochlorid zum Produkt der allgemeinen Formel I mit Z in der Bedeutung der Hydroxyiminogruppierung N~OH umsetzt.

Die Freisetzung der 3-Keto-Funktion unter gleichzeitiger Wasserabspaltung und Ausbildung der 4(5)-Doppelbindung erfolgt durch Behandlung mit Säure oder einem sauren Ionenaustauscher. Die sauere Behandlung erfolgt in an sich bekannter Weise, indem man das entsprechende 5α-Hydroxy-3-ketal in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis vorhandene Schutzgruppen entfernt sind und gegebenenfalls Wasser abgespalten ist. Die Umsetzung, die bei Temperaturen von 0 bis 100 °C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Im allgemeinen wird die Schutzgruppenentfernung und Wasserabspaltung in einem Reaktionsschritt bewerkstelligt, indem man das entsprechende 5α-Hydroxy-3-ketal bzw. 5-en-3-Ketal in stark saurem Medium eine gewisse Zeit lang reagieren läßt. Genauso gut ist es aber erfindungsgemäß möglich, die Schutzgruppenentfernung und Wasserabspaltung in zwei voneinander getrennten Reaktionsschritten durchzuführen, indem zuerst durch eine kürzere Behandlung des entsprechenden 5α-Hydroxy-3-ketals in mäßig saurem Medium zunächst die entsprechende 5α-Hydroxy-3-keto-Verbindung gewonnen und gegebenenfalls isoliert wird. Die 5α-Hydroxy-3-keto-Verbindung wird dann durch weiteres Einwirkenlassen von Säure unter Wasserabspaltung in die 3-Keto-4-en-Verbindung überführt.

Zur Herstellung der Ausgangsprodukte der allgemeinen Formel II sind verschiedene Synthesewege möglich. Um zu Verbindungen sowohl der allgemeinen Formeln II′ als auch II″ (unter Verbindungen der Formeln II′ und II″ sollen diejenigen verstanden werden, die letztlich zu den Endverbindungen der Formel I′ bzw. den überbrückten Verbindungen der Formel I″ führen) zu gelangen, ist erfindungsgemäß die im nachstehenden Reaktionsschema I angegebene Synthese geeignet.

Mit dem angegebenen, breit anwendbaren Verfahren gelingt insbesondere auch die Herstellung von Ausgangsverbindungen der Formel II, worin $R^{1'}$ und/oder $R^{3'}$ und/oder $R^{4'}$ eine oder mehrere C-C-Mehrfachbindungen aufweisende Substituenten sein können, da diese erst nach der Hydrierung von VII → VIII eingeführt werden.

Die Herstellung der Startverbindung III ist in der EP-A-0 127 864 beschrieben; $R^2$ und K haben die in Formel II angegebene Bedeutung.

Durch Grignard-Addition (Tetrahedron Letters 1979, 2051) von Arylhalogeniden

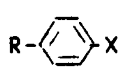

und 2-Halogen-arylmethylhalogeniden.

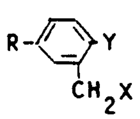 (X = Cl, Br; Y = F, Cl, Br, J;

R = $-OCH_3$, $N(CH_3)_2$, $OSiR_3^{IV}$),

wobei die Additionsprodukte im zuletzt genannten Fall noch einer reduktiven Cyclisierungsreaktion (Birch-Reduktion mit $Li/NH_3$ oder Reduktion mit $Bu_3SnH$) unterworfen werden, gelangt man zu Verbindungen der allgemeinen Formel IV (Beispiel 1; Herstellung der Ausgangsverbindung).

Reaktionsschema I

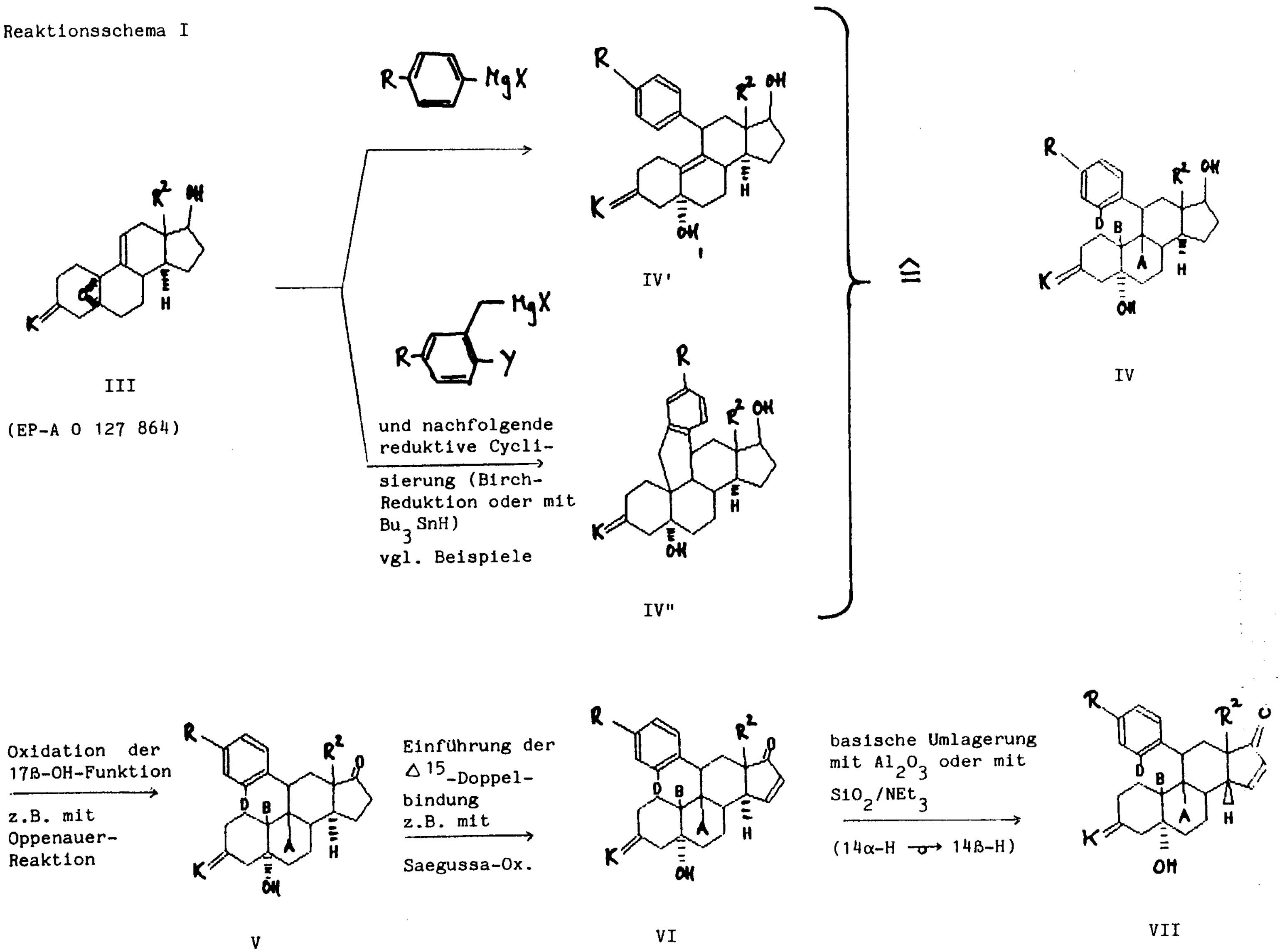


III (EP-A 0 127 864)

IV'

IV"

IV

V

VI

VII

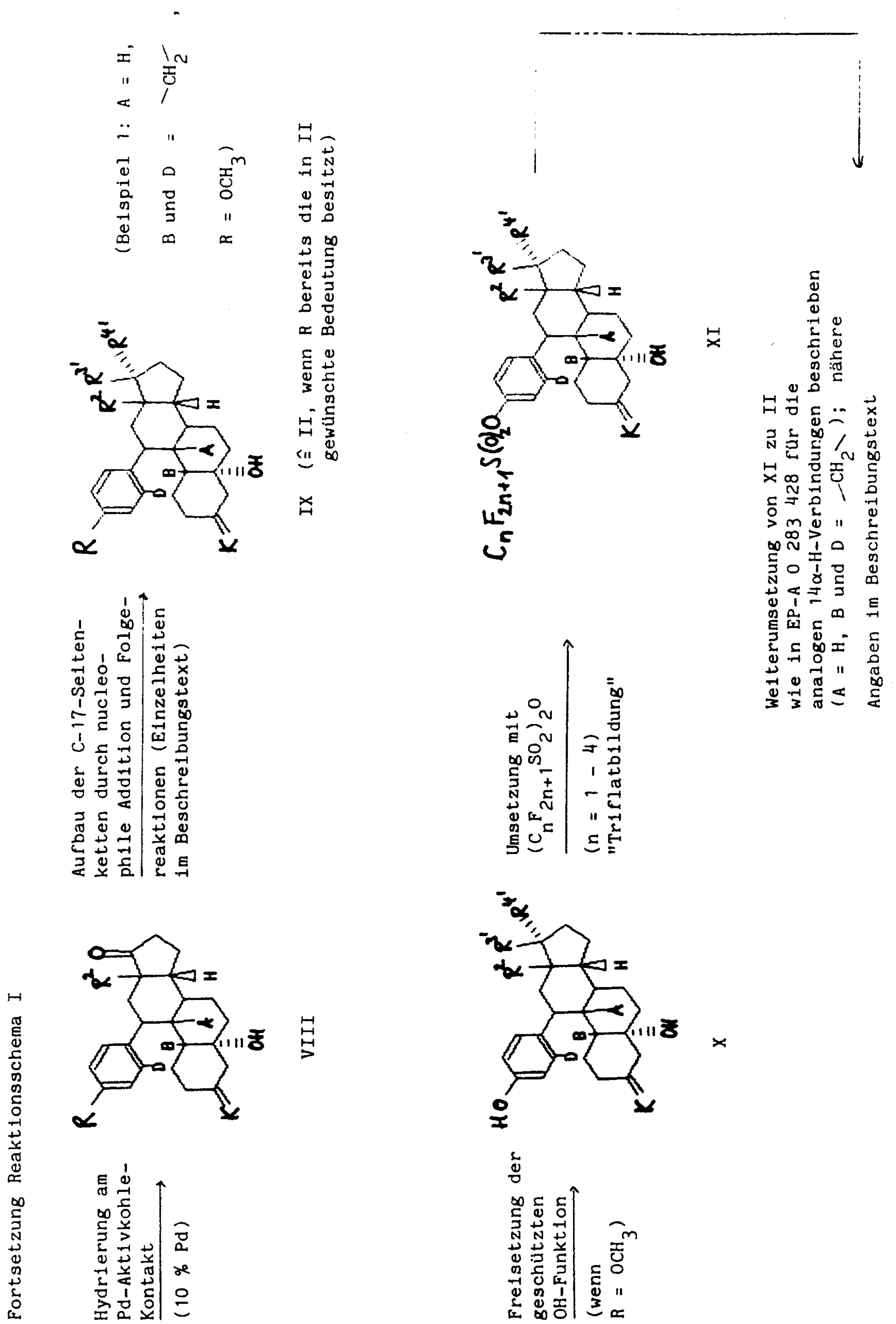


Oxidation der C-17-Hydroxy- zur 17-Ketogruppe (z.B. Oppenauer-Oxidation führt zu Verbindungen der allgemeinen Formel V.

Die Zwischenverbindungen der Formel VI mit einem ungesättigten D-Ring sind zum Beispiel durch modifizierte Saegusa-Oxidation [Tetrahedron 42 (1986) 2971] der entsprechenden Enolverbindungen der 17-Ketons zugänglich. Zum Beispiel ist der Trimethylsilylenolether durch Überführung des 17-Ketons mit

Lithiumdiisopropylamid in Tetrahydrofuran in das korrespondierende Enolat und Abfang durch Trimethylchlorsilan darstellbar (Synthesis 1983, 1).

Durch basische Behandlung von VI, beispielsweise durch Rühren mit basischem Aluminiumoxid oder mit Kieselgel/Triethylamin in einem inerten Lösungsmittel, lagert diese in das entsprechende Steroid mit $\beta$-ständigem 14H der Formel VII um. Die $\Delta^{15}$-Doppelbindung wird nachfolgend durch Hydrierung am Palladium/Aktivkohle-Kontakt (10 % Pd) wieder beseitigt.

Die Einführung der Substituenten $R^{3'}$ und $R^{4'}$ erfolgt nach den üblichen Verfahren des C-17-Seitenkettenaufbaus durch nucleophile Addition an das 17-Keton III und Folgereaktionen ("Terpenoids and Steroids", Specialist Periodical Report, The Chemical Society, London, Vokl. 1-12).

Die Einführung des Substituenten -C≡C-Y als $R^{3'}$, wobei Y die oben angegebene Bedeutung hat, erfolgt mit Hilfe einer Verbindung der allgemeinen Formel MC≡C-Y′, in der Y′ der mit einer Schutzgruppe, wie zum Beispiel Trimethylsilyl oder tert.-Butyldimethylsilyl, geschützte Rest Y oder aber im Fall, wenn Y eine Alkylgruppe mit 1 - 4 C-Atomen ist, Y′ selbst der Rest Y ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen bzw. -propan in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17$\alpha$-(3-Hydroxyprop-1-inyl)-17$\beta$-hyrdoxyderivat oder mit metallierten Derivaten des 3-Hydroxypropins, zum Beispiel mit 1-Lithium-3-(tetrahydropyran-2′-yloxy)-prop-1-in-1-id, zum 17-[3-(Tetrahydropyran-2′-yloxy)-prop-1-inyl]-17$\beta$-hydroxyderivat, die anschließend zu den 17-(3-Hydroxypropyl- bzw. Hydroxypropenyl)-17$\beta$-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134; und H.O. House: Modern Synthetic Reactions 1972, Seite 19). Als desaktivierte Edelmetallkatalysatoren kommen beispielsweise 10% Palladium auf Bariumsulfat in Gegenwart eines Amins oder 5 % Palladium auf Calciumcarbonat unter Zusatz von Blei(II)acetat infrage. Die Hydrierung wird nach der Aufnahme von einem Äquivalent Wasserstoff abgebrochen.

Die Verbindung mit der E-Konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. A. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2′-yloxy)-prop-1(E)-en (J. Org. Chem. 40 2265) oder 1-Lithium-3-(tetrahydropyran-2′-yloxy)-prop-1(Z)-en (Synthesis 1981, 999). Homologe können auf diese Art ebenfalls eingeführt werden.

Die Einführung von 3-Hydroxypropan sowie -butan in 17-Stellung kann ebenfalls direkt durch Umsetzung des 17-Ketons mit metallierten Derivaten von 3-Halogen-propanolen bzw. -butanolen - wobei die Hydroxygruppe im Metallierungsschritt als Alkoholat (Tetrahedron Letters 1978, 3013) oder als geschützte Funktion (J. Org. Chem. 37. 1947) vorliegt - zu der 17-(3-Hydroxypropyl)-17$\beta$-hydroxy-Verbindung bzw. zu der an der terminalen Hydroxygruppe geschützten Verbindung erfolgen. Als Schutzgruppen kommen zum Beispiel die Ethoxyethyl-, Tetrahydropyranyl- und Methoxymethyl-Gruppen infrage.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem. Ber. 113 (1984), 1184 bzw. US-Patent 4,600,538 beschriebenen Methoden.

Werden Endprodukte der Formel I gewünscht mit $R^3/R^4$ in der Bedeutung von

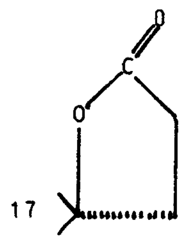

oder

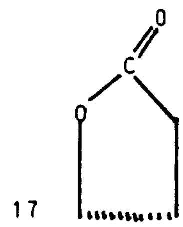

.

so wird die 17-(3-Hydroxypropyl)-Verbindung in an sich bekannter Weise oxidiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite (Compt. rend. 267 [1968] 900). Cyclisierung der 17-(3-Hydroxypropyl)- bzw. 17-(3-Hydroxypropenyl)-Verbindung nach bekannten Verfahren ergibt den entsprechenden gesättigten oder ungesättigten cyclischen Ether.

Zur Einführung der Gruppierungen

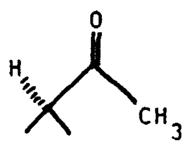

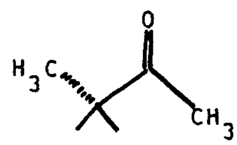

wird das 17-Keton mit Tosylmethylisocyanid (Chem. Ind. 1972 213) in die 17-Nitrilverbindung (Tetrahedron 31 (1975), 2151) überführt, das direkt mit Methyllithium oder Methylmagnesiumbromid in die 17-Acetylverbindung umgewandelt werden kann, welche nach Enolisierung mit K-tert.-Butylat in Tetrahydrofuran und Umsetzung mit Methyljodid die gewünschte 17α-Methyl-17β-acylgruppierung liefert. Diese Sequenz Methyladdition an das Nitril und anschließende Alkylierung kann auch im umgekehrter Folge ausgeführt werden.

Vorhandene freie Hydroxy- bzw. Hydroxy-, Mercapto- und/oder Aminogruppen können in an sich bekannter Weise alkyliert oder acyliert werden.

Sulfide und/oder Dialkylamine können durch geeignete Oxidationsmittel (zum Beispiel Wasserstoffperoxid bzw. Persäuren) in die gewünschten Sulfoxide (n = 1), N-oxide (n = 1) [siehe z.B. Kontakte (Darmstadt) 1986, 3, S. 12] bzw. Sulfone (n = 2) überführt werden.

Verbindungen mit einem Dialkylamin-Substituenten in $R^{1'}$ können durch Reaktion mit Bromcyan in aprotischen Lösungsmitteln wie zum Beispiel Dioxan, Benzol oder Toluol bei erhöhter Temperatur (Amin-Abbau nach Braun) analog den zum Beispiel in Org. Reactions 7, 198 (1953), K.W. Bentley, Techniques of Organic Chemistry 11, 773 (1963) und Houben-Weyl, 5/4, 151 (1960) angegebenen Vorschriften in guter Ausbeute in die entsprechenden (N-Cyan-N-alkylaminoaryl)-Derivate überführt werden.

Diese werden je nach der letztlich gewünschten Bedeutung von $R^1$ im Endprodukt in an sich bekannter Weise zu den entsprechenden Dialkylamin-Verbindungen (zum Beispiel mit Diisobutylaluminiumhydrid in Toluol zu den N-Formyl-N-alkkylaminophenyl-Zwischenprodukten und anschließend mit Lithiumaluminiumhydrid) bzw. N-H-N-alkyl-Verbindungen reduziert (zum Beispiel mit Lithiumaluminiumhydrid oder mit Lithium in flüssigem Ammoniak). Die letzteren werden anschließend gewünschtenfalls in literaturbekannter Weise acyliert und gegebenenfalls anschließend in bekannter Weise mit zum Beispiel Lithiumaluminiumhydrid zum neuen Dialkylaminderivat reduziert (s. DE 36 23 038).

Exemplarisch für einen Vertreter der Verbindungen der allgemeinen Formel IX ist die Darstellung von 3,3-(2,2-Dimethyltrimethylendioxy)-11β,19-(4-methoxy-o-phenylen)-17-[3-(tetrahydropyran-2-yloxy)-propyl]-14β-androstan-5α,17α-diol in Beispiel 1d) beschrieben.

Durch Freisetzung der geschützten Hydroxygruppe und Umsetzung mit Perfluoralkylsulfonsäureanhydrid [Alkyl mit 1 bis 4 Kohlenstoffatomen; P. J. Stang, M. Hanack und L. R. Subramanian, Synthesis 85

(1982)] gelangt man über Verbindungen der allgemeinen Formel X zu den "Schlüsselverbindungen" der allgemeinen Formel XI (vgl. ebenfalls Beispiel 1).

In den Verbindungen der allgemeinen Formel XI kann wie in der EP-A 0 283 428 für die analogen (überbrückten) 14α-H-Steroide beschrieben, die Trifluoralkylsulfonsäureabgangsgruppe ausgetauscht werden, um zu Verbindungen der allgemeinen Formel II zu gelangen, worin $R^{1'}$ eine andere Bedeutung gemäß vorliegender Erfindung hat als R.

Bei der Umsetzung der Trifluoralkylsulfonatverbindungen der Formel XI zu Verbindungen der Formel II wird entweder so vorgegangen, daß in einer übergangsmetallkatalysierten Reaktion (vorzugsweise Pd°) die Perfluoralkylsulfonatabgangsgruppe unter im wesentlichen fast gleichzeitiger Substitution durch den gewünschten Substituenten $R^{1'}$ oder dessen Vorstufe, beispielsweise durch Reagierenlassen mit Tributylvinyl-, Tributylallyl- oder Tributyl-1-ethoxyvinyl-zinn oder mit einem anderen, den gewünschten Substituenten enthaltenden Trialkylzinnderivat, verdrängt wird (J. E. McMurry and S. Mohanraj, Tetrahedron Letters, 24, No. 27, S. 2723-2726, 1983; X. Lu and J. Zhu, Communications, S. 726-727, 1987; Q.-Y, Chen und Z.-Y. Yang, Tetrahedron Letters 27, No. 10, S. 1171-1174, 1986; S. Cacchi, P.G. Ciattini, E. Morera und G. Ortar, Tetrahedron Letters, 27, No. 33, S.3931-3934, 1986; A.M. Echavarren und J.K. Stille, J. Am. Chem. Soc. 1987, 109, S. 5478-5486), oder es wird aus der Perfluoralkylsulfonat-Verbindung intermediär eine entsprechende Triorganylstannyl-, vorzugsweise Tri-n-alkylstannyl-Verbindung hergestellt [J.K. Stille, Angew. Chem. 98 (1986), S. 504-519]. Diese wird anschließend in einer Eintopf-Reaktion übergangsmetallkatalysiert mit einem halogen-, vorzugsweise brom- oder jodsubstituierten carbocyclischen oder heterocyclischen Aromaten [Y. Yamamoto, Y. Azuma, H. Mitoh, Communications, S. 564-565, 1986; T.J. Bailey, Tetrahedron Letters, 27, No. 37, S. 4407-4410, 1986], der gegebenenfalls noch weitere Substituenten tragen kann, zu einer Verbindung der allgemeinen Formel II umgesetzt; der Phenylrest weist darin dann die gewünschte bzw. einen Vorläufer der gewünschten Substitution auf. Die intermediär auftretenden, beispielsweise mit Hexabutyldizinn herstellbaren Tri-n-alkylstannyl-Verbindungen (Alkyl = Butyl) können gewünschtenfalls isoliert werden.

Zu den Verbindungen der Formel II', worin $R^{1'}$ allerdings dann nicht für einen Cycloalkenylrest stehen kann, gelangt man auch über die nachstehend im Reaktionsschema II angegebene Reaktionssequenz. Da ein Cycloalkenylrest die Hydrierung von XVI → XVII nicht unbeschadet überstehen würden, sind entsprechende erfindungsgemäße 11β-(4-Cycloalkenyl)-phenylsteroide auf diese Weise nicht darstellbar.

Zur Veranschaulichung der im Reaktionsschema II angegebenen Variante gibt Beispiel 2 stellvertretend die Herstellung von 17α-Hydroxy-17-(1-propinyl)-11β-[4-(2-thiazolyl)-phenyl]-14β-estra-4,9-dien-3-on wieder.

Als weiteres Beispiel für die Umlagerung eines 14α-H-Steroids in das entsprechende 14β-H-Steroid zeigt Beispiel 3 die Herstellung von 11β-(4-Acetylphenyl)-17α-hydroxy-17-(1-propinyl)-14β-estra-4,9-dien-3-on und aus Beispiel 4 geht anhand der Darstellung von 11β-(4-Acetylphenyl)-17α-hydroxy-17-(2-propinyl)-14β-estra-4,9-dien-3-on hervor, wie die 2-Propinylfunktion als C-17-Seitenkette eingeführt wird.

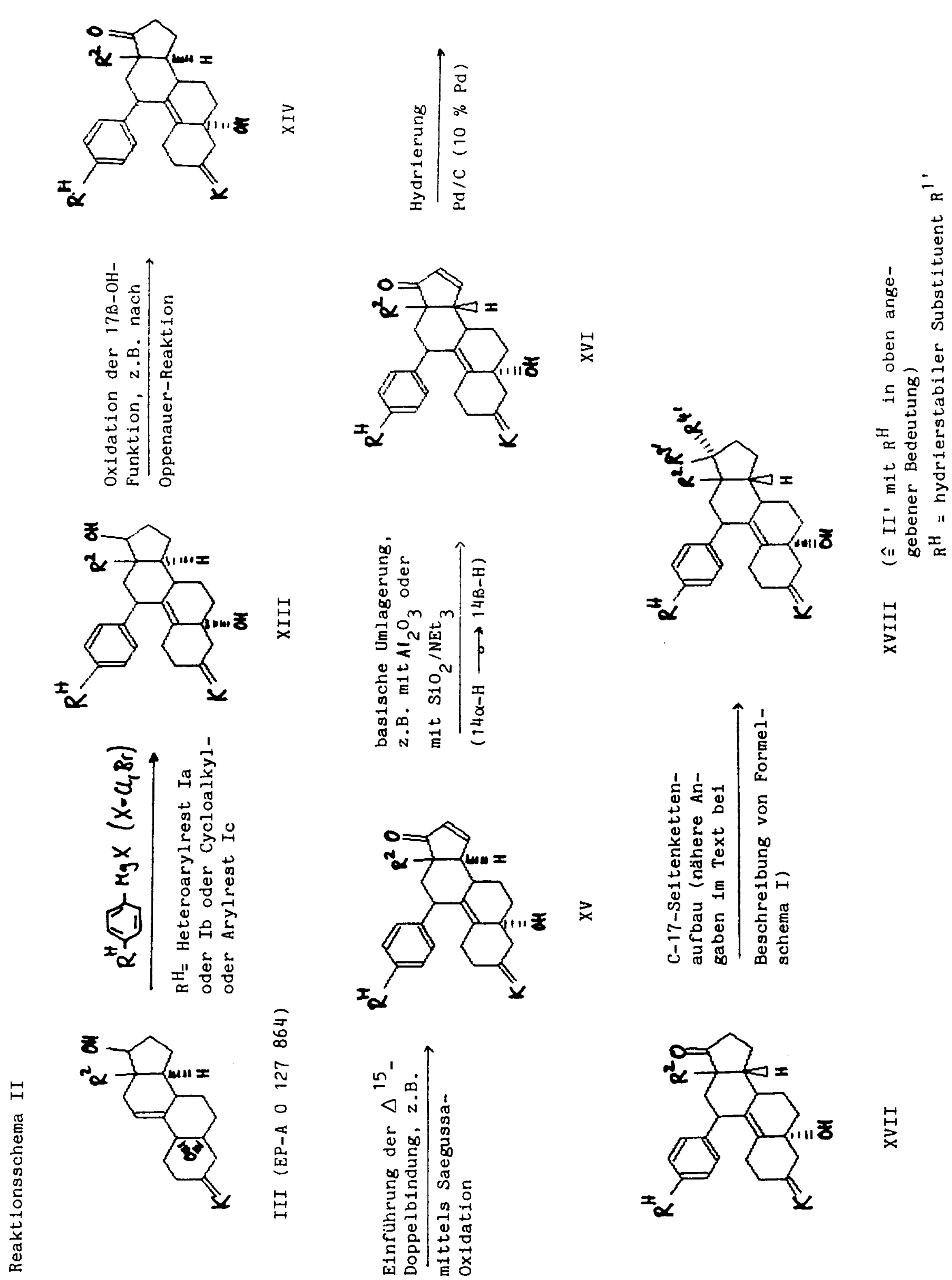


Um zu den überbrückten Ausgangsverbindungen der Formel II" zu gelangen, ist es - abweichend von Reaktionsschema I -, wenn der für den Rest R stehende Substituent hydrierungsstabil ist (wenn diese also keine C-C-Mehrfachbindungen aufweist) genausogut möglich, aus einer Verbindung der Formel IV"

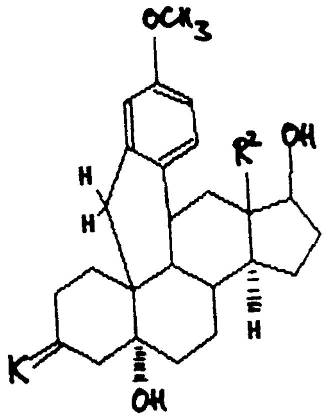

(IV")

durch Abspaltung der O-Methylgruppe [Tetrahedron Lett. 1327, (1970)] die freie OH-Verbindung zu erzeugen, diese mit Trifluormethansulfonsäureanhydrid zur entsprechenden Trifluormethansulfonatverbindung umzusetzen und diese anschließend in gewünschter Weise wie bereits angegeben in der 4-Stellung des o-Phenylenringes zu funktionalisieren und erst dann anschließend die 17-OH-Funktion, beispielsweise durch Oppenauer-Oxidation in die 17-Keto-Funktion zu überführen. Eine so erhaltene Verbindung der allgemeinen Formel V" (vgl. EP-A- 0 283 428)

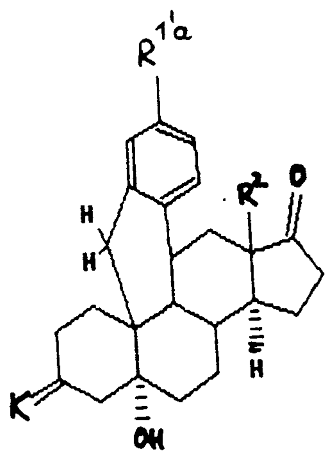

(V"),

worin $R^{1'a}$ für alle unter $R^{1'}$ angegebenen Substituenten steht, mit Ausnahme von $OR^{III}$, $NR^{I}R^{II}$ und der in $R^{1'}$ enthaltenen nicht-hydrierungsstabilen Substituenten [Hydrierungsbedingungen: $H_2$-Normaldruck, Pd/C (10 % Pd)], wird - wie bereits angegeben - durch Einführung der $\Delta^{15}$-Doppelbindung, basische Umlagerung der 14α-H- in die 14β-H-Verbindung und Aufbau der C-17-Seitenketten, in eine Verbindung der allgemeinen Formel $II^{''a}$

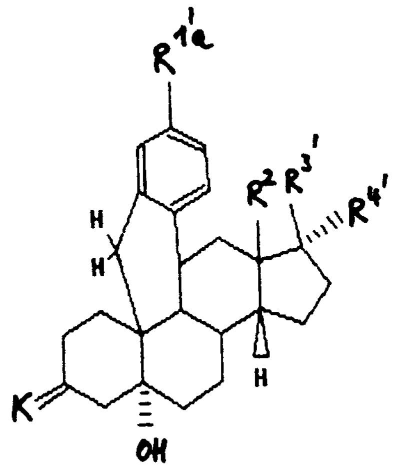

(II"a)

überführt.

Die nach Schutzgruppenabspaltung und Dehydratisierung erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40 °C in die Oxime (Formel I mit X in der Bedeutung der Hydroxyiminogruppierung N OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]nonen-5 (DBN) und 1,5-diazabicyclo[5.4.0]undecen-5 (DBU), wobei Pyridin bevorzugt ist.

Zur Entfernung der 3-Oxogruppe zu einem Endprodukt der allgemeinen Formel I mit X in der Bedeutung von 2 Wasserstoffatomen kann z.B. nach der in DOS 28 05 490 angegebenen Vorschrift durch Thioketalisierung und anschließende reduktive Spaltung erfolgen.

## Beispiel 1

17-(3-Hydroxypropyl)-17α-hydroxy-11β-19-(4-methoxy-o-phenylen)-14β-androst-4-en-3-on (9)

a)

3,3-(2,2-Dimethyltrimethylendioxy)-5α-hydroxy-11β,19-(4-methoxy-o-phenylen)-14-androsten-17-on (4)

---

9,50 g 11β,19-(4-Methoxy-o-phenylen)-5α-hydroxy-3,3-(2,2-dimethyltrimethylendioxy)-androstan-17-on (1) (Herstellung dieser Ausgangsverbindung ist im Anschluß an Beispiel 1 beschrieben) werden zu einer Lösung von 57,4 mmol Lithiumdiisopropylamid in 220 ml absolutem Tetrahydrofuran, gelöst in 80 ml absolutem Tetrahydrofuran, unter Schutzgas bei 0 °C zugetropft. Anschließend wird zum Reaktionsgemisch Chlortrimethylsilan (13.8 ml) zugetropft. Nach 30minütigem Nachrühren wird die Reaktionslösung auf eiskalte gesättigte Natriumhydrogencarbonatlösung gegossen, die wäßrige Phase mit Essigester extrahiert und die organische Phase mit Wasser und gesättigter Ammoniumchloridlösung gewaschen. Nach Trocknen über Natriumsulfat wird die organische Phase im Vakuum eingeengt. Es werden 10,8 g 3,3-(2,2-Dimethyltrimethylendioxy)-11β,19-[4-methoxy-o-phenylen)-17-trimethylsilyloxy-16-androsten-5α-ol (2) roh als gelblicher Schaum isoliert. Dieses Rohprodukt wird in 127 ml absolutem Acetonitril suspendiert und mit 6,4 g Palladium(II)acetat versetzt. Nach 12stündigem Nachrühren bei Raumtemperatur wird das Reaktionsgemisch über Celite abgesaugt, der Filterrückstand mit Essigester nachgewaschen, das Filtrat im Vakuum eingeengt und der Rückstand über Kieselgel (Korngröße 0,2 - 0,5 mm) filtriert. Nach Einengen des Filtrats erhält man 7,1 g 3,3-(2,2-Dimethyltrimethylendioxy)-5α-hydroxy-11β,19-(4-methoxy-o-phenylen)-15-androsten-17-on (3) roh als gelblichen Schaum. Dieses Rohprodukt wird in 2 l einer Mischung aus Hexan/Essigester (1:9) gelöst, mit 100 ml Triethylamin versetzt und anschließend 12 Stunden in Gegenwart von 500 g Kieselgel gerührt. Nach Filtration wird eingeengt und

der Rückstand an 800 g Kieselgel mit einem Gemisch aus Hexan/Essigester/Triethylamin (80:19:1) chromatographiert. Man erhält als 1. Fraktion 3,47 g der obigen Verbindung 4.
$[\alpha]_D^{22} = +40.8°$ (C: 0.5, $CHCl_3$).
Als 2. Fraktion werden 2,41 g eines Gemisches (1:1, nach DC) aus 3 und der obigen Verbindung 4 eluiert.
b)

3,3-(2,2-Dimethyltrimethylendioxy)-5α-hydroxy-11β,19-(4-methoxy-o-phenylen)-14β-androstan-17-on (6)

---

Unter Schutzgas werden 6,22 ml Diisopropylamin in 200 ml absolutem Tetrahydrofuran bei -10 °C vorgelegt und mit 30,5 ml einer 1,6 M n-Butyllithiumlösung (Hexan) versetzt. Es wird 30 Minuten bei -5 °C nachgerührt und anschließend 3,11 g der unter a) dargestellten Verbindung 4, gelöst in 60 ml absolutem Tetrahydrofuran, zugetropft. Das Reaktionsgemisch wird 15 Minuten bei -5 °C gerührt und dann mit 11,2 ml Trimethylchlorsilan tropfenweise versetzt. Nach 15 Minuten wird auf -70 °C gekühlt und 5,4 ml Fluorwasserstoff-Pyridin-Komplex zugetropft. Anschließend wird das Reaktionsgemisch 2 Stunden bei -60 °C nachgerührt und dann auf gesättigte Natriumhydrogencarbonatlösung gegossen. Die wäßrige Phase wird mit Essigester extrahiert, gewaschen (NaCl-Lösung), über Natriumsulfat getrocknet und im Vakuum eingeengt. Es werden 3,1 g 3,3-(2,2-Dimethyltrimethylendioxy)-5α-hydroxy-11β,19-(4-methoxy-o-phenylen)-14β-androst-15-en-17-on (5) roh als gelblicher Schaum erhalten. Dieses Rohprodukt wird in 200 ml Ethanol gelöst und mit 369 mg Palladium/Kohle (10 % Pd) hydriert. Filtration über Celite und Entfernen des Lösungsmittels im Vakuum liefert nach Chromatographie mit Hexan/Essigester an Kieselgel 2,18 g der obigen Verbindung 6.
$[\alpha]_D^{22} = +55°$ (C: 0,51, $CHCl_3$)
c)

3,3-(2,2-Dimethyltrimethylendioxy)-11β,19-(4-methoxy-o-phenylen)-17-[3-(tetrahydropyran-2-yloxy)-1-propinyl]-14β-androstan-5α,17α-diol (7)

---

Aus 6,18 g 3-(Tetrahydropyran-2-yloxy)-1-propin in 215 ml absolutem Tetrahydrofuran und 27,5 ml einer 1,6 M Lösung von n-Butyllithium (Hexan) stellt man bei -10 °C die lithiumorganische Verbindung her und tropft dazu bei -10 °C eine Lösung von 2,18 g der unter b) erhaltenen Verbindung 6 in 45 ml absolutem Tetrahydrofuran. Man rührt erst 1 Stunde bei 0 °C, danach über Nacht bei Raumtemperatur. Man gießt in Eiswasser und extrahiert mit Essigester. Das Rohprodukt wird an neutralem Aluminiumoxid mit Hexan/Essigester chromatographiert. Als Hauptfraktion erhält man 2,65 g der obigen Verbindung 7 als weißen Schaum.
d)

3,3-(2,2-Dimethyltrimethylendioxy)-11β,19-(4-methoxy-o-phenylen)-17-[3-(tetrahydropyran-2-yloxy)-propyl]-14β-androstan-5α,17α-diol (8)

---

Wie unter b) beschrieben werden aus 2.65 g der unter c) hergestellten Verbindung 7 in 150 ml Ethanol und 265 mg Palladium/Kohle (10 % Pd) nach Hydrierung 2,53 g der obigen Verbindung 8 erhalten.
e) 2,53 g der unter d) hergestellten Verbindung 8 werden in 125 ml Aceton gelöst und mit 6 ml 4n Salzsäure versetzt. Nach 4stündigem Nachrühren bei Raumtemperatur wird das Reaktionsgemisch auf gesättigte Natriumhydrogencarbonatlösung gegossen und die wäßrige Phase mit Methylenchlorid extrahiert. Die vereinigten organischen Phasen werden über Natriumsulfat getrocknet und im Vakuum eingeengt. Der Rückstand wird über Kieselgel chromatographiert. Es werden 1,16 g der Titelverbindung

9 erhalten.
$[\alpha]_D^{22}$ = 82,6° (C: 0,5, $CHCl_3$).
f)

3,3-(2,2-Dimethyltrimethylendioxy)-17-[3-(tetrahydropyran-2-yloxy)-propyl]-11β,19-(4-trifluormethylsulfonyloxy-o-phenylen)-14β-androstan-5α,17α-diol (11)

2 g der nach Beispiel 1d) hergestellten Methoxyverbindung 8 werden in 31 ml absolutem Dimethylformamid gelöst und unter Schutzgas mit 658 mg Natriummethanthiolat versetzt. Das Reaktionsgemisch wird 3 Stunden unter Rückfluß erhitzt, anschließend auf Raumtemperatur abgekühlt und dann auf 200 ml Eiswasser gegossen. Es wird solange bei Raumtemperatur nachgerührt, bis das Rohprodukt als feste Substanz ausgeflockt ist. Anschließend wird es abgesaugt und am Vakuum getrocknet. Es werden 1,90 g 3,3-(2,2-Dimethyltrimethylendioxy)-11β,19-(4-hydroxy-o-phenylen)-17-[3-(tetrahydropyran-2-yloxy)-propyl]-14β-androstan-5α,17α-diol (10) roh erhalten. Dieses Rohprodukt wird in 45 ml absolutem Methylenchlorid gelöst und mit 2,04 g 4-Dimethylaminopyridin versetzt. Unter Schutzgas wird die Lösung auf -70 °C gekühlt und langsam durch Zutropfen mit 0,7 ml Trifluormethansulfonsäureanhydrid, gelöst in 6 ml absolutem Methylenchlorid, versetzt. Nach 1stündigem Nachrühren bei -60 °C wird das Reaktionsgemisch auf gesättigte Natriumhydrogencarbonatlösung gegossen und die wäßrige Phase mit Methylenchlorid extrahiert. Die vereinigten organischen Phasen werden mit gesättigter Natriumchloridlösung gewaschen, über Natriumsulfat getrocknet und am Vakuum eingeengt. Man erhält nach Chromatographie des Rückstandes an Kieselgel 1,73 g der obigen Verbindung 11 als gelblichen Schaum.
IR (KBr): 1210 und 1420 $cm^{-1}$ Triflat.

**Herstellung der Ausgangsverbindung (1)**

11β,19-(4-Methoxy-o-phenylen)-5α-hydroxy-3,3-(2,2-dimethyltrimethylendioxy)-androstan-17-on

a) 19-(4-Methoxy-2-chlorphenyl)-3,3-(2,2-dimethyltrimethylendioxy)-9(11)-androsten-5α,17β-diol
5,4 g Magnesiumspäne werden bei Raumtemperatur unter Schutzgas in 45 ml absolutem Dieethylether vorgelegt und zunächst mit 0,55 ml 2-Chlor-5-methoxy-benzylchlorid und dann vorsichtig mit 0,4 ml 1,2-Dibromethan versetzt. Nach erfolgtem Start der Reaktion wird anschließend die restliche Menge (19,4 ml) des 2-Chlorbenzylchlorids, gelöst in 135 ml absolutem Diethylether, über 40 Minuten hinweg zugetropft, ohne daß die Innentemperatur im Reaktionsgefäß über 30 °C steigt. Nach erfolgter Bildung des Grignard-Reagenzes wird das Reaktionsgemisch auf 0 °C abgekühlt und 5α,10α-Epoxy-3,3-(2,2-dimethyltrimethylendioxy)-9(11)-estren-17β-ol (15 g), gelöst in 80 ml absolutem Tetrahydrofuran, langsam zugetropft. Nach 1stündigem Nachrühren bei Eisbadtemperatur wird über Nacht das Reaktionsgemisch langsam auf Raumtemperatur erwärmt und anschließend auf verdünnte Ammoniumchloridlösung gegossen. Die wäßrige Phase wird mehrmals mit Essigester extrahiert. Die vereinigten organischen Phasen werden mit Natriumchloridlösung neutralgewaschen, mit Natriumsulfat getrocknet und im Vakuum eingeengt. Der Rückstand wird an Aluminiumoxid (neutral, Stufe III) chromatographiert. Es werden 15,5 g obiger Verbindung erhalten.
b) 11β,19-(4-Methoxy-o-phenylen)-3,3-(2,2-dimethyltrimethylendioxy)-androstan-5α,17β-diol
Bei -65 °C werden 600 ml wasserfreier Ammoniak unter Feuchtigkeitsausschluß in den Reaktionskolben einkondensiert und mit 970 mg frisch geschnittenen Lithiumspänen versetzt. Unmittelbar nach Auftreten der charakteristischen Blaufärbung wird eine Lösung von 15 g des unter a) erhaltenen Produkts in 450 ml absolutem Tetrahydrofuran derart zugetropft, daß ein Wechselspiel zwischen Entfärbung der Reaktionslösung und Blaufärbung entsteht. Nach erfolgter Zugabe wird das überschüssige Lithium durch Zutropfen von Ethanol beseitigt, der überwiegende Teil des Ammoniaks durch Abdampfen entfernt und das Reaktionsgemisch auf Wasser gegossen. Die wäßrige Phase wird mit Essigester extrahiert. Die vereinigten organischen Phasen werden anschließend mit Natriumchloridlösung gewaschen, mit Natriumsulfat getrocknet und im Vakuum eingeengt. Es werden 13,9 g Rohprodukt isoliert. Chromatographie an Aluminiumoxid (neutral, Stufe III) ergibt 11,6 g obiger Verbindung.
$[\alpha]_D^{22}$ = +21,1° ($CHCl_3$); c = 0,52)
Fp.: = 223 - 224 °C (Diisopropylether)

c) 11β,19-(4-Methoxy-o-phenylen)-5α-hydroxy-3,3-(2,2-dimethyltrimethylendioxy)-androstan-17-on

Zu einem Gemisch aus 43,2 ml Pyridin und 384 ml Methylenchlorid werden bei 0 °C portionsweise 14,2 g Chromtrioxid zugegeben. Anschließend werden 11 g des nach b) erhaltenen Steroids, gelöst in 75 ml Methylenchlorid, langsam bei der gleichen Temperatur zum Reaktionsgemisch zugetropft und dieses weitere 2 Stunden bei Eisbadtemperatur nachgerührt. Nach Beendigung des Rührens läßt man die festen Reaktionsbestandteile sich absetzen, dekantiert die überstehende Phase ab und wäscht den Niederschlag mehrmals kräftig mit Methylenchlorid aus. Die vereinigten organischen Phasen werden durch Waschen mit wäßriger 0,5 m Kaliumhydroxidlösung von restlichen organischen Bestandteilen befreit, mit Wasser neutral gewaschen, über Natriumsulfat getrocknet und im Vakuum eingeengt. Es werden 9 g rohes 11β,19-(4-Methoxy-o-phenylen)-5α-hydroxy-3,3-(2,2-dimethyltrimethylendioxy)-androstan-17-on isoliert, dessen Reinheit für weitere Reaktionen ausreichend ist. 500 mg werden für analytische Zwecke durch Chromatographie an Aluminiumoxid (neutral, Stufe III) gereinigt. Es werden 443 mg des gewünschten Produkts als weißer Schaum isoliert.

$[\alpha]_D^{22}$ = +33° ($CHCl_3$; c = 0,55)

Fp.: = 235 - 238 °C

**Beispiel 2**

17α-Hydroxy-17-(1-propinyl)-11β-[4-(2-thiazolyl)-phenyl]-14β-estra-4,9-dien-3-on (28)

a)

3,3-(2,2-Dimethyltrimethylendioxy)-11β-[4-(2-thiazolyl)-phenyl]-9-estren-5α,17β-diol (21)

13,4 g Magnesiumspäne werden unter Schutzgas in 200 ml absolutem Tetrahydrofuran vorgelegt und bei 50 °C mit 3,5 ml Dibromethan versetzt. Nach erfolgter Reaktion wird eine Lösung von 97,8 g 1-Chlor-4-(2-thiazolyl)-benzol [Darstellung anch Lit.: Tetrahedron Letters 27, 4407 (1986)] in 250 ml absolutem Tetrahydrofuran langsam zugetropft. Es wird 4 Stunden bei 70 °C nachgerührt. Nach vollständiger Umsetzung wird die Grignard-Lösung auf 0 °C abgekühlt und mit 1,43 g Kupfer-(I)-chlorid versetzt. Nach 30 Minuten wird eine Lösung von 37,4 g 5α, 10α-Epoxy-3,3-(2,2-dimethyltrimethylendioxy)-9(11)-estren-17-ol (EP-A 0 127 864) in 300 ml absolutem Tetrahydrofuran zugetropft. Nach Zugabe wird das Reaktionsgemisch für weitere 30 Minuten bei 0 °C und 15 Minuten bei Raumtemperatur gerührt und dann auf Eiswasser gegossen. Die wäßrige Phase extrahiert man mit Essigester, vereinigt die organischen Phasen und wäscht sie mit gesättigter Kochsalzlösung. Nach Trocknen über Natriumsulfat und Einengen am Vakuum chromatographiert man den so erhaltenen Rückstand an Aluminiumoxid mit einem Gemisch aus Essigester/Hexan. Es werden 40.1 g der obigen Verbindung 21 erhalten.

b)

3,3-(2,2-Dimethyltrimethylendioxy)-5α-hydroxy-11β-[4-(2-thiazolyl)-phenyl]-9-estren-17-on (22)

40,1 g der unter 2a) hergestellten vErbindung 21 werden in 650 ml Toluol gelöst, mit 20,1 g Aluminiumisopropylat und 140 ml Cyclohexanon versetzt und 2 Stunden am Wasserabscheider unter Rückfluß erhitzt. Man läßt abkühlen, tropft ca. 400 ml gesättigte Natriumhydrogencarbonatlösung zu und saugt über Celite ab. Das Filtrat wird gewaschen (NaCl-Lösung), getrocknet und im Vakuum eingeengt. Der so erhaltene Rückstand wird an Aluminiumoxid mit einem Gemisch aus Essigester/Hexan chromatographiert. Man erhält 25,9 g der obigen Verbindung 22.

c)

3,3-(2,2-Dimethyltrimethylendioxy)-5α-hydroxy-11β-[4-(2-thiazolyl)-phenyl]-9,15-estradien-17-on (24)

Analog Beispiel 1a) werden aus 25,9 g der unter 2b) dargestellten Verbindung 22 145 mmol Lithiumdiisopropylamid und 34,8 ml Chlortrimethylsilan 29,3 g 3,3-(2,2-Dimethyltrimethylendioxy)-11β-[4-(2-thiazolyl)-phenyl]-17-trimethylsilyloxy-9,16-estradien-5α-ol (23) roh als gelblicher Schaum erhalten. Dieses Rohprodukt wird in 250 ml absolutem Acetonitril suspendiert und mit 15,0 g Palladium(II)acetat versetzt. Die Aufarbeitung erfolgt wie im Beispiel 1a) beschrieben. Es werden 21,1 g der obigen Verbindung 24 als Rohprodukt isoliert.

d)

3,3-(2,2-Dimethyltrimethylendioxy)-5α-hydroxy-11β-[4-(2-thiazolyl)-phenyl]-14β-estra-9,15-dien-17-on (25)

21,1 g der unter Beispiel 2c) hergestellten Verbindung 24, gelöst in 2,0 l Essigester, werden mit 140 ml Triethylamin versetzt und in Gegenwart von 950 g Kieselgel 24 Stunden gerührt. Nach Filtration wird im Vakuum eingeengt und der Rückstand an 2 kg Kieselgel mit einem Gemisch aus Hexan/Essigester/Triethylamin (80:19:1) chromatographiert. Es werden 13,1 g der obigen Verbindung 25 erhalten.

e)

3,3-(2,2-Dimethyltrimethylendioxy)-5α-hydroxy-11β-[4-(2-thiazolyl)-phenyl]-14β-estr-9-en-17-on (26)

Eine Lösung von 13,1 g der unter 2d) hergestellten Verbindung in 450 ml Ethanol wird mit 1,3 g Palladium/Kohle (10 % Pd) hydriert. Filtration über Celite und Entfernen des Lösungsmittels im Vakuum liefert nach Chromatographie mit Hexan/Essigester an Aluminiumoxid 9,3 g der obigen Verbindung 26.

f)

3,3-(2,2-Dimethyltrimethylendioxy)-17β-(1-propinyl)-11β-[4-(2-thiazolyl)-phenyl]-14β-estr-9-en-5α,17α-diol (27)

200 ml absolutes Tetrahydrofuran werden durch 30minütiges Einleiten von Methylacetylen bei 0 °C gesättigt. Anschließend tropft man bei 0 °C bis 5 °C 28,5 ml einer 1,6 M Lösung von n-Butyllithium in Hexan zu, rührt nach Zugabe 15 Minuten nach und gibt dann eine Lösung von 1,23 g der unter Beispiel 2e) hergestellten Verbindung in 20 ml absolutem Tetrahydrofuran tropfenweise hinzu. Das Reaktionsgemisch wird nach erfolgter Zugabe 1 Stunde nachgerührt, auf Eiswasser gegossen und die wäßrige Phase mit Essigester extrahiert. Die vereinigten organischen Phasen werden über Natriumsulfat getrocknet und im Vakuum eingeengt. Der Rückstand wird an Aluminiumoxid mit einem Gemisch aus Essigester/Hexan chromatographiert. Es werden 823 mg obiger Verbindung 27 erhalten.

g) 823 mg der unter Beispiel 2f) hergestellten Verbindung werden in 13 ml 70 % wäßriger Essigsäure gelöst und 1 Stunde bei 50 °C unter Schutzgas gerührt. Nach dem Abkühlen gießt man in Eiswasser, neutralisiert durch Zugabe von gesättigter Natriumhydrogencarbonatlösung und extrahiert mit Essigester. Die vereinigten organischen Phasen werden über Natriumsulfat getrocknet und im Vakuum eingeengt.

Den Rückstand chromatographiert man mit einem Gemisch aus Hexan/Essigester an Kieselgel. Es werden 310 mg der Titelverbindung 28 als weißer Schaum isoliert.
$^1$H-NMR ($CDCl_3$ + Py.$d_5$) δ: 0,91 (s; 3H, H-$CH_3$), 1,89 (s; 3H, H-$CH_3$),
4,5 (m; 1H, 11-H), 5,69 (s; 1H, 4-H),
7,30; 7,80 (jeweils d, J = 3Hz; 2H, -S-CH=CH-N-)
7,50; 7,75 (AA'BB'-System, J = 9Hz; HH,arH).

**Beispiel 3**

11β-(4-Acetylphenyl)-17α-hydroxy-17-(1-propinyl)-14β-estra-4,9-dien-3-on

750 mg 11β-{4-[1,1-(2,2-Dimethyltrimethylendioxy)-ethyl]-phenyl}-3,3-(2,2-dimethyltrimethylendioxy)-17β-(1-propinyl)-14β-estr-9-en-5α,-17α-diol werden in 12 ml 70 % wäßriger Essigsäure gelöst und 1 Stunde bei 50 °C unter Schutzgas gerührt. Nach dem Abkühlen gießt man in Eiswasser, neutralisiert durch Zugabe von gesättigter Natriumhydrogencarbonatlösung und extrahiert mit Essigester. Die vereinigten organischen Phasen werden über Natriumsulfat getrocknet und im Vakuum eingeengt. Den Rückstand chromatographiert man mit einem Gemisch aus Hexan/Essigester an Kieselgel. Es werden 300 mg der Titelverbindung als weißer Schaum isoliert.
$[\alpha]_D^{22}$ = 242° ($CHCl_3$; c = 0.500).

**Die Herstellung des Ausgangsmaterials erfolgt auf folgendem Wege:**

a) Unter Schutzgas werden 10 ml Diisopropylamin in 290 ml absolutem Tetrahydrofuran bei -10 °C vorgelegt und mit 50 ml einer 1,6 m n-Butyllithiumlösung (Hexan) versetzt. Es wird eine halbe Stunde bei 0 °C nachgerührt, dann wieder auf -10 °C gekühlt und 13,6 g 11β-{4-[1,1-(2,2-dimethyltrimethylendioxy)-ethyl]-phenyl}-5α-hydroxy-3,3-(2,2-dimethyltrimethylendioxy)-9-estren-17-on (Herstellung nach Europäische Patentanmeldung 86101548.5, Publikations-Nr. 190759, Beispiel 6), gelöst in 150 ml absolutem Tetrahydrofuran, zugetropft. Nach erfolgter Zugabe rührt man 15 Minuten nach und tropft dann 17,2 ml Trimethylchlorsilan zu. Anschließend wird das Reaktionsgemisch auf eiskalte gesättigte Natriumhydrogencarbonatlösung gegossen und die wäßrige Phase mit Essigester extrahiert. Die vereinigten organischen Phasen werden mehrmals mit gesättigter Ammoniumchloridlösung gewaschen und im Vakuum eingeengt. Den Rückstand kristallisiert man aus 50 ml Acetonitril. Es werden 13,2 g 17-Trimethylsilyloxy-11β-{4-[1,1-(2,2-dimethyltrimethylendioxy)-ethyl]-phenyl}-3,3-(2,2-dimethyltrimethylendioxy)-9,16-estradien-5α-ol erhalten.
b) In 150 ml absolutem Acetonitril werden 4,27 g Palladium(II)-acetat vorgelegt und mit 12,12 g der unter a) hergestellten Verbindung versetzt. Das Reaktionsgemisch wird 16 Stunden bei Raumtemperatur gerührt, anschließend über Kieselgel filtriert und der Filterrückstand gut mit Methylenchlorid gewaschen. Die organische Phase wird im Vakuum eingeengt und der Rückstand an Kieselgel chromatographiert. Es werden 10 g 11β-{4-[1,1-(2,2-Dimethyltrimethylendioxy)-ethyl]-phenyl}-5α-hydroxy-3,3-(2,2-dimethyltrimethylendioxy)-9,15-estradien-17-on als weißer Schaum isoliert.
c) 17,3 g 11β-{4-[1,1-(2,2-Dimethyltrimethylendioxy)-ethyl]-phenyl}-3,3-(2,2-dimethyltrimethylendioxy)-5α-hydroxy-estra-9,15-dien-17-on, gelöst in 1,73 l Essigester, werden mit 120 ml Triethylamin versetzt und in Gegenwart von 800 g Kieselgel 24 Stunden gerührt. Nach Filtration wird im Vakuum eingeengt und der Rückstand an 1,73 g kg Kieselgel mit einem Gemisch aus Hexan/Essigester/Triethylamin (49:49:2) chromatographiert. Es werden 10,4 g 11β-{4-[1,1-(2,2-Dimethyltrimethylendioxy)-ethyl]-phenyl}-3,3-(2,2-dimethyltrimethylendioxy)-5α-hydroxy-14β-estra-9,15-dien-17-on erhalten.
d) Eine Lösung von 3,0 g der unter 3c) hergestellten Verbindung in 150 ml Ethanol wird mit 300 mg Palladium/Kohle (10 % Pd) hydriert. Filtration über Celite und Entfernen des Lösungsmittels im Vakuum liefert nach Chromatographie mit Hexan/Essigester an Aluminiumoxid 2,34 g 11β-{4-[1,1-(2,2-Dimethyltrimethylendioxy)-ethyl]-phenyl}-3,3-(2,2-dimethyltrimethylendioxy)-5α-hydroxy-14β-estr-9-en-17-on.
Schmelzpunkt: 145 - 148 °C.
e) 200 ml absolutes Tetrahydrofuran werden durch 30minütiges Einleiten von Methylacetylen bei 0 °C gesättigt. Anschließend tropft man bei 0 °C bis 5 °C 28,5 ml einer 1.6 M Lösung von n-Butyllithium in Hexan zu, rührt nach Zugabe 15 Minuten nach und gibt dann eine Lösung von 1,32 g der unter 3d) hergestellten Verbindung in 20 ml absolutem Tetrahydrofuran tropfenweise hinzu. Das Reaktionsgemisch wird nach erfolgter Zugabe 60 Minuten nachgerührt, auf Eiswasser gegossen und die wäßrige Phase mit Essigester extrahiert. Die vereinigten organischen Phasen werden über Natriumsulfat getrocknet und im Vakuum eingeengt. Der Rückstand wird an Aluminiumoxid (neutral, Stufe III) mit einem Gemisch aus

Essigester/Hexan chromatographiert. Es werden 800 mg 11β-{4-[1,1-(2,2-Dimethyltrimethylendioxy)-ethyl]-phenyl}-3,3-(2,2-dimethyltrimethylendioxy)-17β-(1-propinyl)-14β-estr-9-en-5α, 17α-diol erhalten.

$^1$H-NMR($CD_2Cl_2$) δ: 0,57 (s; 3H, H-$CH_3$), 0,81 (s; 3H, H-$CH_3$),
0,90 (s; 3H, H-$CH_3$), 0,99 (s; 3H, 18-H),
1,22 (s; 3H, H-$CH_3$), 1,48 (s; 3H, H-$CH_3$),
1,91 (s; 3H, H-$CH_3$).

**Beispiel 4**

11β-(4-Acetylphenyl)-17α-hydroxy-17-(2-propinyl)-14β-estra-4,9-dien-3-on

470 mg 11β-(4-Acetylphenyl)-17α-hydroxy-17-(3-trimethylsilyl)-2-propinyl)-14β-estra-4,9-dien-3-on in 24 ml Tetrahydrofuran werden mit 2,35 ml einer 1 M Lösung von Tetrabutylammoniumfluorid in Tetrahydrofuran versetzt und 5 Minuten bei Raumtemperatur gerührt. Man gießt auf Wasser und extrahiert mit Essigester. Die vereinigten organischen Phasen werden mit Wasser gewaschen, über Natriumsulfat getrocknet und eingeengt. Den Rückstand chromatographiert man mit einem Gemisch aus Hexan/Essigester an Kieselgel. Es werden 280 mg der Titelverbindung als Schaum isoliert.
$[\alpha]_D^{22}$ = 166° ($CHCl_3$; c = 0.500).

**Die Herstellung des Ausgangsmaterials erfolgt auf folgendem Wege:**

a) Zu einer Lösung von 2,33 g 1-(Trimethylsilyl)-1-propin in 100 ml absolutem Tetrahydrofuran werden bei -5 °C 13,6 ml einer 1,6 M Lösung von n-Butyllithium in Hexan getropft. Man rührt nach Zugabe 1 Stunde bei dieser Temperatur nach, kühlt auf -78 °C und gibt dann eine Lösung von 1,20 g der unter 3d) dargestellten Verbindung in 13 ml Tetrahydrofuran tropfenweise hinzu. Das Reaktionsgemisch wird über Nacht bei Raumtemperatur nachgerührt, auf Eiswasser gegossen und die wäßrige Phase mit Essigester extrahiert. Die vereinigten organischen Phasen werden über Natriumsulfat getrocknet und eingeengt. Der Rückstand wird mit einem Gemisch aus Hexan/Essigester an Kieselgel chromatographiert. Es werden 940 g 11β-{4-[1,1-(2,2-Dimethyltrimethylendioxy)-ethyl]-phenyl}-3,3-(2,2-dimethyltrimethylendioxy)-17-(3-trimethylsilyl-2-propinyl)-14β-estr-9-en-5α,17α-diol als Schaum erhalten.

b) Wie unter Beipiel 3 beschrieben werden aus 940 mg der unter 4a) hergestellten Verbindung und 13 ml 70 % wäßriger Essigsäure 486 mg 11β-(4-Acetylphenyl)-17α-hydroxy-17-(3-trimethylsilyl-2-propinyl)-14β-estra-4,9-dien-3-on erhalten.
$[\alpha]_D^{22}$ = 150° ($CHCl_3$; c = 0.500).

Allgemeine Vorschrift 1 zur Herstellung der Verbindungen der allgemeinen Formel I'' durch saure Spaltung der Verbindungen der allgemeinen Formel II (Tabelle 1)

Eine Lösung von x g Steroid in y ml Aceton wird mit z ml 4N-Salzsäure versetzt und a Minuten bei t °C unter Argon gerührt. Anschließend gießt man auf gesättigte Natriumhydrogencarbonat-Lösung und extrahiert mit Methylenchlorid. Die vereinigten organischen Phasen werden mit gesättigter Kochsalzlosung gewaschen, über Natriumsulfat getrocknet und im Vakuum eingeengt. Durch Chromatographie an Kieselgel mit einem Gemisch aus Ethylacetat/Hexan erhält man aus dem Rohprodukt b g der gewünschten Verbindung der allgemeinen Formel I'.

Allgemeine Vorschrift 2 zur Herstellung der Verbindungen der allgemeinen Formel I' durch saure Spaltung der Verbindungen der allgemeinen Formel II (Tabelle 2)

Eine Losung von x g Steroid in y ml 70%iger Essigsäure wird z Minuten bei t °C gerührt. Anschließend gießt man auf Eiswasser, neutralisiert durch Zugabe von wäßriger Ammoniak-Lösung und extrahiert mit Methylenchlorid. Analog obiger Aufarbeitung erhält man a g der gewünschten Verbindung der allgemeinen Formel I'.

**BEISPIEL 5**

17α-Hydroxy-17-(3-hydroxypropyl)-11β,19-[4-vinyl-o-phenylen]-14β-androst-4-en-3-on (32) (Tabelle 1)

a) 3,3-(2,2-Dimethyltrimethylendioxy)-17-[3-(tetrahydropyran-2-yloxy)-propyl]-11β,19-(4-tri-n-butylstannyl-o-phenylen)-14β-androstan-5α,17α-diol (29)

4,62 g 3,3-(2,2-Dimethyltrimethylendioxy)-17-[3-(tetrahydropyran-2-yloxy)-propyl-11β,19-(4-trifluormethylsulfonyloxy-o-phenylen)-14β-androstan-5α,17α-diol (Verbindung 11, Beispiel 1f) werden unter Schutzgas in 160 ml absolutem Dioxan vorgelegt, mit 9,7 ml Hexabutyldizinn, 290 mg Tetrakis-(triphenylphosphin)-palladium(0) und 810 mg Lithiumchlorid versetzt und anschließend 30 Minuten unter Rückfluß erhitzt. Nach Filtration über Celite wird am Vakuum eingeengt und der Rückstand an Aluminiumoxid mit einem Gemisch aus Essigester/Hexan chromatographiert. Es werden 5,7 g der obigen Verbindung erhalten.

b) 3,3-(2,2-Dimethyltrimethylendioxy)-17-[3-(tetrahydropyran-2-yloxy)-propyl]-11β,19-[4-iod-o-phenylen)-14β-androstan-5α,17α-diol (30)

3,2 g der unter a) hergestellten Verbindung werden in 80 ml Methylenchlorid gelöst und bei 0 °C unter Argon mit 905 mg Iod-Kristallen portionsweise versetzt. Nach 30 Minuten wird das Reaktionsgemisch mit gesättigter Natriumthiosulfat- und gesättigter Kochsalz-Lösung gewaschen. Nach dem Trocknen über Natriumsulfat, Einengen im Vakuum und säulenchromatographischer Reinigung über Aluminiumoxid werden 1,9 g der obigen Verbindung erhalten.

c) 3,3-(2,2-Dimethyltrimethylendioxy)-17-[3-(tetrahydropyran-2-yloxy)-propyl] 11β,19-(4-vinyl-o-phenylen)-14β-androstan-5α,17α-diol (31)

Variante 1

1,9 g der unter b) hergestellten Verbindung werden unter Schutzgas in 80 ml absolutem Toluol vorgelegt, mit 1,03 g Tributylvinylzinn und 298 mg Tetrakis-(triphenylphosphin)-palladium(0) versetzt und 30 Minuten unter Rückfluß erhitzt. Nach Einengen am Vakuum und Reinigung an Kieselgel werden 480 mg der obigen Verbindung erhalten.

Variante 2

0,99 g 3,3-(2,2-Dimethyltrimethylendioxy)-17-[3-(tetrahydropyran-2-yloxy)-propyl]-11β,19-(4-trifluoromethylsulfonyloxy-o-phenylen)-14β-androstan-5α,17α-diol (11) werden unter Schutgas in 20 ml absolutem Dimethylformamid vorgelegt, mit 0,54 g Tributylvinylzinn, 158 mg Tetrakis-(triphenylphosphin)-palladium(0) und 113 mg Lithiumchlorid versetzt und 30 Minuten unter Rückfluß erhitzt. Nach Einengen am Vakuum und Reinigung an Kieselgel werden 640 mg der obigen Verbindung erhalten.

d) Nach der allgemeinen Vorschrift 1 wird die Titelverbindung 32 erhalten (Tabelle 1)

**BEISPIEL 6**

17α-Hydroxy-17-(3-hydroxypropyl)-11β,19-(4-ethyl-o-phenylen)-14β-androst-4-en-3-on (34) (Tabelle 1)

a) 3,3-(2,2-Dimethyltrimethylendioxy)-17-[3-(tetrahydropyran-2-yloxy)-propyl]-11β,19-(4-ethyl-o-phenylen)-14β-androstan-5α,17α-diol (33)

640 mg der unter Beispiel 5c) hergestellten Verbindung in 50 ml absolutem Tetrahydrofuran werden nach Zusatz von 140 mg Palladium/$CaCO_3$ (5% Pd) unter Normaldruck hydriert. Nach Filtration über Celite und säulenchromatographischer Reinigung an Kieselgel werden 550 mg der obigen Verbindung 33 isoliert.

b) Nach der allgemeinen Vorschrift 1 wird die Titelverbindung 34 erhalten (Tabelle 1).

**BEISPIEL 7**

17α-Hydroxy-17-(3-hydroxypropyl)-11β,19-[4-(3-pyridyl)-o-phenylen]-14β-androst-4-en-3-on(36) (Tabelle 1)

a) 3,3-(2,2-Dimethyltrimethylendioxy)-17-[3-(tetrahydropyran-2-yloxy)-propyl] 11β,19-[4-(3-pyridyl)-o-phenylen]-14β-androstan-5α,17α-diol (35)

1,65 g der unter Beispiel 5a) hergestellten Verbindung werden unter Schutzgas in 45 ml absolutem Toluol vorgelegt, mit 2,9 g 3-Brompyridin und 212 mg Tetrakis-(triphenylphosphin)-palladium(0) versetzt

und 15 Stunden unter Rückfluß erhitzt. Nach Einengen am Vakuum und Reinigung an Kieselgel werden 800 mg der obigen Verbindung erhalten.
b) Nach der allgemeinen Vorschrift 1 wird die Titelverbindung 36 erhalten.

**BEISPIEL 8**

17α-Hydroxy-17-(3-hydroxypropyl)-11β,19-[4-(3-thienyl)-o-phenylen]-14β-androst-4-en-3-on(38) (Tabelle 1)

a) 3,3-(2,2-Dimethyltrimethylendioxy)-17-[3-(tetrahydropyran-2-yloxy)-propyl] 11β,19-[4-(3-furyl)-o-phenylen]-14β-androstan-5α,17α-diol (37)
1,74 g der unter Beispiel 5a) hergestellten Verbindung werden unter Schutzgas in 40 ml absolutem Toluol vorgelegt, mit 3,15 g 3-Bromthiophen und 224 mg Tetrakis-(triphenylphosphin)-palladium(0) versetzt und 15 Stunden unter Rückfluß erhitzt. Nach Einengen am Vakuum und Reinigung an Kieselgel werden 720 mg der obigen Verbindung erhalten.
b) Nach der allgemeinen Vorschrift 1 wird die Titelverbindung 38 erhalten.

**BEISPIEL 9**

17α-Hydroxy-17-(3-hydroxypropyl)-11β,19-(4-dimethylamino-o-phenylen)-14β-androst-4-en-3-on(51) (Tabelle 1)

a) 3,3-(2,2-Dimethyltrimethylendioxy)-5α-hydroxy-11β,19-(4-dimethylamino-o-phenylen)-14β-androsten-17-on (45)
18,6 g 11β,19-(4-Dimethylamino-o-phenylen)-5α-hydroxy-3,3-2,2-dimethyltrimethylendioxy)-androstan-17-on (42) (Herstellung dieser Ausgangsverbindung ist im Anschluß an Beispiel 9 beschrieben) werden zu einer Lösung von 109,4 mmol Lithiumdiisopropylamid in 450 ml absolutem Tetrahydrofuran, gelöst in 150 ml absolutem Tetrahydrofuran, unter Schutzgas bei 0 °C zugetropft. Anschließend wird zum Reaktionsgemisch Chlortrimethylsilan (26,4 ml) zugetropft. Nach 30 minütigem Nachrühren wird die Reaktionslosung auf eiskalte gesättigte Natriumhydrogencarbonatlösung gegossen, die wäßrige Phase mit Essigester extrahiert und die organische Phase mit Wasser und gesättigter Ammoniumchloridlösung gewaschen. Nach Trocknen über Natriumsulfat wird die organische Phase im Vakuum eingeengt. Es werden 20,1 g 3,3-(2,2-Dimethyltrimethylendioxy)-11β,19-(4-dimethylamino-o-phenylen)-17-trimethylsilyloxy-16-androsten-5α-ol (43) roh als gelblicher Schaum isoliert. Dieses Rohprodukt wird in 250 ml absolutem Acetonitril suspendiert und mit 11,6 g Palladium-(II)acetat versetzt. Nach 12 stündigem Nachrühren bei Raumtemperatur wird das Reaktionsgemisch über Celite abgesaugt, der Filterrückstand mit Essigester nachgewaschen, das Filtrat im Vakuum eingeengt und der Rückstand über Kieselgel (Korngröße 0,2-0,5 mm) filtriert. Nach Einengen des Filtrats erhält man 7,0 g 3,3-(2,2-Dimethyltrimethylendioxy)-5α-hyroxy-11β,19-(4-dimethylamino-o-phenylen)-15-androsten-17-on (44) roh als gelblichen Schaum. Dieses Rohprodukt wird in 1 l einer Mischung aus Hexan/Essigester (1:9) gelöst, mit 72 ml Triethylamin versetzt und anschließend 72 Stunden in Gegenwart von 400 g Kieselgel gerührt. Nach Filtration wird eingeengt und der Rückstand an 800 g Kieselgel mit einem Gemisch aus Hexan/Essigester/Triethylamin (80:19:1) chromatographiert. Man erhält als 1. Fraktion 5,36 g der obigen Verbindung 45. Als 2. Fraktion werden 0,93 g eines Gemisches (3:1, nach DC) aus 44 und der obigen Verbindung 45 eluiert.
b) 3,3-(2,2-Dimethyltrimethylendioxy)-5α-hydroxy-11β,19-(4-dimethylamino-o-phenylen)-14β-androstan-17-on (47)
Unter Schutzgas werden 6,01 ml Diisopropylamin in 180 ml absolutem Tetrahydrofuran bei -10 °C vorgelegt und mit 29,5 ml einer 1,6 M n-Buthyllithiumlösung (Hexan) versetzt. Es wird 30 Minuten bei -5 °C nachgerührt und anschließend 3,1 g der unter a) dargestellten Verbindung 45, gelöst in 50 ml absolutem Tetrahydrofuran, zugetropft. Das Reaktionsgemisch wird 15 Minuten bei -5 °C gerührt und dann mit 10 ml Trimethylchlorsilan tropfenweise versetzt. Nach 15 Minuten wird auf -70 °C gekühlt und 5,2 ml Fluorwasserstoff-Pyridin-Komplex zugetropft. Anschließend wird das Reaktionsgemisch 10 Stunden bei -30 °C nachgerührt und dann auf gesättigte Natriumhydrogencarbonatlösung gegossen. Die wäßrige Phase wird mit Essigester extrahiert, gewaschen (NaCl-Lösung), über Natriumsulfat getrocknet und im Vakuum eingeengt. Es werden 3,0 g 3,3-(2,2-Dimethyltrimethylendioxy)-5α-hydroxy-11β,19-(4-dimethylamino-o-phenylen)-14β-androst-15-en-17-on (46) roh als gelblicher Schaum erhalten. Dieses Rohprodukt wird in 120 ml Tetrahydrofuran/Ethanol (1:1) gelöst und mit 500 mg Palladium/Calciumcarbonat (5% Pd) hydriert. Filtration über Celite und Entfernen des Lösungsmittels im

Vakuum liefert nach Chromatographie mit Hexan/Essigester an Kieselgel 2,4 g der obigen Verbindung 47.

c) 3,3-(2,2-Dimethyltrimethylendioxy)-11β,19-(4-dimethylamino-o-phenylen)-17-[3 (tetrahydropyran-2-yloxy)-1-propinyl]-14β-androstan-5α,17α,-diol (48)

Aus 9,94 g 3-(Tetrahydropyran-2-yloxy)-1-propin in 170 ml absolutem Tetrahydrofuran und 45,0 ml einer 1,6M Lösung von n-Butyllithium (Hexan) stellt man bei -10 °C die lithiumorganische Verbindung her und tropft dazu bei -10 °C eine Lösung von 2.40 g der unter b) erhaltenen Verbindung 47 in 45 ml absolutem Tetrahydrofuran. Man rührt erst 1 Stunde bei 0 °C nach, danach über Nacht bei Raumtemperatur. Man gießt in Eiswasser und extrahiert mit Essigester. Das Rohprodukt wird an neutralem Aluminiumoxid mit Hexan/Essigester chromatographiert. Als Hauptfraktion erhält man 2,14 g der obigen Verbindung 48 als weißen Schaum.

d) 3,3-(2,2-Dimethyltrimethylendioxy)-11β,19-(4-dimethylamino-o-phenylen)-17-[3 -(tetrahydropyran-2-yloxy)-propyl]-14β-androstan-5α,17α-diol (49)

Wie unter b) beschrieben werden aus 2,14 g der unter c) hergestellten Verbindung 48 in 140 ml Ethanol/Tetrahydrofuran (1:1) und 430 mg Palladium/Calciumcarbonat (5% Pd) nach Hydrierung 1,79 g der obigen Verbindung 49 erhalten.

e) 17-(3-Hydroxypropyl)-17α,5α-dihydroxy-11β,19-(4-dimethylamino-o-phenylen)-14β-androst-4-en-3-on (50)

1,77 g der unter d) hergestellten Verbindung 49 werden in 90 ml Aceton gelöst und mit 4.4 ml 4N Salzsäure versetzt. Nach 16-stündigem Nachrühren bei 0 °C wird das Reaktionsgemisch auf gesättigte Natriumhydrogencarbonatlösung gegossen und die wäßrige Phase mit Methylenchlorid extrahiert. Die vereinigten organischen Phasen werden über Natriumsulfat getrocknet und im Vakuum eingeengt. Der Rückstand wird über Kieselgel chromatographiert. Es werden 0,98 g der obigen Verbindung 50 erhalten. $^1$H-NMR($CDCl_3$) δ:0,55(3H,s,18-H); 2,93(6H,s,$NMe_2$); 3,68(2H,m,$CH_2OH$); 6,4-7,3(3H,3m, Protonen am Aromaten).

f) 0,98 g der unter e) hergestellten Verbindung 50 werden in 50 ml Aceton gelöst und mit 2,5 ml 4N Salzsäure versetzt. Nach 4 stündigem Nachrühren bei 25 °C wird wie unter e) beschrieben aufgearbeitet. Es werden 0,67 g der Titelverbindung 51 erhalten.

**Herstellung der Ausgangsverbindung (42)**

11β,19-(4-Dimethylamino-o-phenylen)-5α-hydroxy-3,3-(2,2-dimethyltrimethylendioxy)-androstan-17-on

a) 19-(4-Dimethylamino-2-chlorphenyl)-3,3-(2,2-dimethyltrimethylendioxy)-9(11)-androsten-5α,17β-diol (40)

7,72 g Magnesiumspäne werden bei Raumtemperatur unter Schutzgas in 50 ml absolutem Diethylether vorgelegt und mit 0,7 ml 1,2-Dibromethan versetzt. Nach erfolgtem Start der Reaktion wird anschließend 64,5 g 2-Chlor-5-dimethylamino-benzylchlorid gelöst in 700 ml absolutem Diethylether, über 40 Minuten hinweg zugetropft, ohne daß die Innentemperatur im Reaktionsgefäß über 30 °C steigt. Nach erfolgter Bildung des Grignard-Reagenzes wird 5α,10α-Epoxy-3,3-(2,2-dimethyltrimethylendioxy)-9(11)-estren-17β-ol (23,6 g), gelöst in 200 ml absolutem Tetrahydrofuran, langsam zugetropft. Nach 1 stündigem Nachrühren bei Eisbadtemperatur wird über Nacht nachgerührt und anschließend auf verdünnte Ammoniumchloridlösung gegossen. Die wäßrige Phase wird mehrmals mit Essigester extrahiert. Die vereinigten organischen Phasen werden mit Natriumchloridlösung neutralgewaschen, mit Natriumsulfat getrocknet und im Vakuum eingeengt. Der Rückstand wird an Aluminiumoxid (neutral, Stufe III) chromatographiert. Es werden 33,0 g obiger Verbindung erhalten.

b) 11β,19-(4-Dimethylamino-o-phenylen)-3,3-(2,2-dimethyltrimethylendioxy)-androstan-5α,17β-diol (41)

Bei -65 °C werden 790 ml wasserfreier Ammoniak unter Feuchtigkeitsausschluß in den Reaktionskolben einkondensiert und mit 2,91 g frisch geschnittenen Lithiumspänen versetzt. Unmittelbar nach Auftreten der charakteristischen Blaufärbung wird eine Lösung von 33 g des unter a) erhaltenen Produkts in 2000 ml absolutem Tetrahydrofuran derart zugetropft, daß ein Wechselspiel zwischen Entfärbung der Reaktionslosung und Blaufärbung entsteht. Nach erfolgter Zugabe wird das überschüssige Lithium durch Zutropfen von Ethanol beseitigt, der überwiegende Teil des Ammoniaks durch Abdampfen entfernt und das Reaktionsgemisch auf Wasser gegossen. Die wäßrige Phase wird mit Essigester extrahiert. Die vereinigten organischen Phasen werden anschließend mit Natriumchloridlösung gewaschen, mit Natriumsulfat getrocknet und im Vakuum eingeengt. Es werden 31,5 g Rohprodukt isoliert. Chromatographie an Kieselgel ergibt 20,0 g obiger Verbindung.

c) 11β,19-(4-Dimethylamino-o-phenylen)-5α-hydroxy-3,3-(2,2-dimethyltrimethylendioxy)-androstan-17-on (42)

Zu 25,1 g der unter b) hergestellten Verbindung in 1000 ml Toluol werden 20,3 g Aluminium-tri-isopropanolat und 147 ml Cyclohexanon gegeben. Man erhitzt 1,5 Stunden unter Rückfluß und destilliert kontinuierlich ca 250 ml ab. Das Reaktionsgemisch wird mit gesättigter Natriumhydrogencarbonat-Lösung versetzt, durch Celite abgesaugt, neutralgewaschen (Natriumchlorid-Lösung) und eingeengt. Chromatographie an Aluminiumoxid mit Hexan/Essigester ergibt 18,6 g der Ausgangsverbindung 42.

**BEISPIEL 10**

11β,19-(4-Dimethylamino-o-phenylen)-4',5'-dihydro-spiro-[14β-androst-4-en-17α,2'-(3'H)-furan]-3-on (52)

404 mg der unter Beispiel 9 dargestellten Verbindung 51 werden in 20 ml Methylenchlorid gelöst und mit 1,2 ml Triethylamin versetzt. Das Gemisch wird auf 0 °C abgekühlt und 500mg p-Toluolsulfonsäurechlorid zugegeben. Nach 60 Minuten bei 0 °C und weiteren 6 Stunden Erhitzen unter Rückfluß wird das Reaktionsgemisch auf gesättigte Natriumhydrogencarbonatlösung gegossen, die wäßrige Phase mit Methylenchlorid extrahiert, die vereinigten organischen Phasen mit gesättigter Kochsalzlösung gewaschen, getrocknet (Natriumsulfat) und im Vakuum eingeengt. Nach Chromatographie des Rohprodukts an Kieselgel werden 370 mg der Titelverbindung 52 isoliert.

$[\alpha]_D^{22}$ = 124,0 ° ($CHCl_3$; c = 0,515)

**BEISPIEL 11**

11β,19-(4-Dimethylamino-o-phenylen)-17α-hydroxy-17-(3-hydroxypropyl)-14β-androsta-1,4-dien-3-on (55)

a) 11β,19-(4-Dimethylamino-o-phenylen)-17α-trimethylsilyloxy-17-(3-trimethylsilyloxypropyl)-3-trimethylsilyloxy-14β-androsta-2,4-dien (53)

1,5 g der nach Beispiel 9f) dargestellten Verbindung 51 gelöst in 45 ml absolutem Tetrahydrofuran werden zu einer Lösung von 33 mmol Lithiumdiisopropylamid in 150 ml absolutem Tetrahydrofuran unter Argon bei 0 °C zugetropft. Anschließend wird zum Reaktionsgemisch Trimethylchlorsilan (7,95 ml) zugetropft. Nach 45minütigem Rühren wird die Reaktionslösung auf kalte gesättigte Natriumhydrogencarbonatlösung gegossen, die wäßrige Phase mit Essigester extrahiert und die organische Phase nacheinander mit Wasser und gesättigter Ammoniumchlorid-Lösung gewaschen. Nach Trocknen über Natriumsulfat wird am Vakuum eingeengt. Es werden 2,48 g des Silyldienolethers 53 als Rohprodukt erhalten.

b) 11β,19-(4-Dimethylamino-o-phenylen)-17α-trimethylsilyloxy-17-(3-trimethylsilyloxypropyl)-14β-androsta-1,4-dien-3-on (54)

2,48 g des unter a) dargestellten Silyldienolethers werden als Rohprodukt in 30 ml absolutem Acetonitril suspendiert und mit 1 g Palladium(II)acetat versetzt. Nach 1stündigem Nachrühren bei Raumtemperatur wird das Reaktionsgemisch über Celite abgesaugt und das Filtrat am Vakuum eingeengt. Es werden 2,31 g des obigen Dienons 54 als Rohprodukt erhalten.

c) 2,31 g des unter b) hergestellten Dienons werden in 30 ml absolutem Methanol gelöst und mit 2,3 g wasserfreiem Kaliumcarbonat versetzt. Unter Schutzgas wird das Gemisch bei Raumtemperatur 1 Stunde nachgerührt, dann auf gesättigte Natriumchloridlösung gegossen und mehrmals mit Methylenchlorid extrahiert. Die vereinigten orgnischen Phasen wäscht man mit gesättigter Natriumchloridlösung, trocknet sie über Natriumsulfat und engt im Vakuum ein. Chromatographie des Rückstandes an Kieselgel mit einem Gemisch aus Essigester/Hexan führen zu 510 mg der Titelverbindung 55.

$^1$H-NMR($CDCl_3$ + Py x $d_5$) δ:0,57(3H,s,18-H); 2,92(6H,s,NMe); 3,70(2H,m,$CH_2$OH); 6,14(1H,s,4-H); 6,15-(1H,d,J = 10Hz,2-H); 7,00(1H,d,J = 10Hz,1-H); 6,4-7,3(3H,3m, Protonen am Aromaten)

**BEISPIEL 12**

11β,19-(4-Dimethylamino-o-phenylen)-17α-hydroxy-17-(1-propinyl)-14β-androsta4,6-dien-3-on (59)

a) 11β,19-(4-Dimethylamino-o-phenylen)-14β-androst-4-en-3,17-dion (56)

Aus 6,0 g der unter Beispiel 9b) beschriebenen Verbindung 47 in 300 ml Aceton und 15 ml 4N Salzsäure werden nach der allgemeinen Vorschrift 1 nach 5 Stunden Rühren bei Raumtemperatur 4,1 g des obigen Enons 56 erhalten.

b) 11β,19-(4-Dimethylamino-o-phenylen)-3-ethoxy-14β-androsta-3,5-dien-17-on (57)

4,1 g des unter a) erhaltenen Produkts werden in einem Gemisch aus 100 ml absolutem Methylenchlorid, 9 ml Ethanol und 10 ml Orthoameisensäuretriethylester vorgelegt und bei 0 °C mit 1,9 g p-Toluolsulfonsäure (Monohydrat) versetzt. Anschließend wird bei Eisbadtemperatur 3 Stunden nachgerührt, dann mit einem Überschuß an Natriumhydrogencarbonatlösung versetzt und die wäßrige Phase mit Methylenchlorid extrahiert. Nach Waschen (Natriumchloridlösung) und Trocknen (Natriumsulfat) der organischen Phasen wird im Vakuum eingeengt. Kristallisation aus Ethanol (ca. 10 ml) ergibt 1,7 g obiger Verbindung.

c) 11β,19-(4-Dimethylamino-o-phenylen)-3-ethoxy-17-(prop-1-inyl)-14β-androsta-3,5-dien-17α-ol (58)

150 ml absolutes Tetrahydrofuran werden bei 0 °C mit Propin gesättigt. Anschließend wird zu dieser Lösung 28,8 ml 1,6 M n-Butyllithium-Lösung (Hexan) langsam zugetropft. Man läßt 30 Minuten nachrühren und tropft eine Lösung von 1,7 g des unter b) dargestellten Produkts in 29 ml absolutem Tetrahydrofuran zum Reaktionsgemisch. Nach 30 Minuten Nachrühren wird auf Wasser gegossen, mit Essigester extrahiert und die vereinigten organischen Phasen mit Natriumchloridlösung gewaschen. Nach Trocknen (Natriumsulfat), Ein-engen im Vakuum und Chromatographie an Aluminiumoxid (neutral, Stufe III) werden 0,99 g obiger Verbindung erhalten.

d) 0,99 g des nach c) erhaltenen Produkts werden bei -50 °C in 100 ml Methylenchlorid vorgelegt, mit 0,34 ml Triethylamin und anschließend mit 409 mg N-Bromsuccinimid versetzt. Es wird 30 Minuten bei -40 °C nachgerührt, danach auf Natriumsulfatlösung gegossen und mit Methylenchlorid mehrmals extrahiert. Nach Trocknen über Natriumsulfat, Einengen im Vakuum und Chromatographie an Kieselgel werden 240 mg leicht verunreinigtes 11β,19-(4-Dimethylamino-o-phenylen)-17α-hydroxy-17-(1-propinyl)-6β-brom-14β-androst-4-en-3-on isoliert. Nach Lösen des Produkts in 3 ml absolutem Dimethylformamid werden unter Argon 91 mg Lithiumbromid und 57 mg Lithiumcarbonat zugefügt und 1 Stunde bei 100 °C gerührt. Nach Abkühlen der Reaktionsmischung auf Raumtemperatur wird sie auf Wasser gegossen, mit 4N Salzsäure neutralisiert, auf 0 °C gekühlt, eine Stunde bei dieser Temperatur nachgerührt und das ausgefallene Steroid abgesaugt. Chromatographie an Kieselgel mit Hexan/Essigester liefert 170 mg der Titelverbindung 59.

$^1$H-NMR($CDCl_3$ + Py x $d_5$) δ:0,55(3H,s,18-H); 1,90(3H,s,Me); 2,90(6H,s,$NMe_2$); 5,75(1H,s,4-H); 6,15-6,25-(2H,m,6-H,7-H); 6,4-7,3(3H,3m,Protonen am Aromaten)

## BEISPIEL 13

11β,19-(4-Dimethylamino-o-phenylen)-17α-hydroxy-17α-methyl-17-(1-propinyl)-14β-androst-4-en-3-on (60)

Aus 90 mg Magnesiumspänen und 0,23 ml Methyliodid in 4 ml absolutem Ether wird wie üblich eine Grignard-Lösung bereitet. Nach Zugabe von 19 mg Kupfer(I)chlorid bei 0 °C wird 30 Minuten nachgerührt und anschließend eine Lösung von 150 mg des unter Beispiel 12d) dargestellten Produkts 59 in 2 ml absolutem Tetrahydrofuran zugetropft. Man läßt 2 Stunden bei 0 °C rühren, gießt auf gesättigte Ammoniumchloridlösung, extrahiert mit Essigester, wäscht mit verdünnter Ammoniak- und danach mit gesättigter Kochsalz-Lösung, trocknet und engt im Vakuum ein. Nach Chromatographie des Rohprodukts an Kieselgel werden 36 mg obiger Titelverbindung 60 isoliert.

$^1$H-NMR($CDCl_3$ + Py x $d_5$) δ:0,56(3H,s,18-H); 0,90(3H,d,J = 7Hz,7α-Me); 1,90(3H,s,Me); 2,92(6H,s,$NMe_2$); 5,82(1H,s,4-H); 6,4-7,3(3H,3m, Protonen am Aromaten)

## BEISPIEL 14

17α-Hydroxy-17-(3-hydroxyprop-(Z)-1-enyl)-11β-[4-(3-pyridyl)-phenyl]-14β-estra-4,9-dien-3-on (69) (Tabelle 2)

a) 3,3-(2,2-Dimethyltrimethylendioxy)-11β-[4-(3-pyridyl)-phenyl]-9-estren-5α,17β-diol (61) Aus 3,57 g Magnesiumspänen in 150 ml absolutem Tetrahydrofuran, 31,2 g 1-Brom-4-(3-pyridyl)-benzol (Darstellung im Anschluß an Beispiel 14) in 100 ml absolutem Tetrahydrofuran, 380 mg Kupfer(I)chlorid und 10,0 g 5α,10α-Epoxy-3,3-(2,2-dimethyltrimethylendioxy)-9(11)-estren-17-ol in 80 ml absolutem Tetrahydrofuran werden, wie unter Beispiel 2a) beschrieben, 10,6 g der obigen Verbindung 61 erhalten.

b) 3,3-(2,2-Dimethyltrimethylendioxy)-5α-hydroxy-11β-[4-(3-pyridyl)-phenyl]-9-estren-17-on (62)

Aus 10,6 g der unter a) dargestellten Verbindung 61 in 180 ml Toluol, 5,26 g Aluminiumisopropylat und 36,6 ml Cyclohexanon werden, wie unter Beispiel 2b) beschrieben, 6,82 g der obigen Verbindung 62 erhalten.

c) 3,3-(2,2-Dimethyltrimethylendioxy)-5α-hydroxy-11β-[4-(3-pyridyl)-phenyl]-9,15-estradien-17-on (64)
Aus 6,82 g der unter b) dargestellten Verbindung 62, 38 mmol Lithiumdiisopropylamid und 9,06 ml Chlortrimethylsilan werden, wie unter Beispiel 2c) beschrieben, 7,5 g 3,3-(2,2-Dimethyltrimethylendioxy)-11β-[4-(3-pyridyl)-phenyl]-17-trimethylsilyloxy-9,16-estradien-5α-ol (63) roh erhalten. Die analoge Umsetzung mit 3,9 g Palladium(II)acetat ergibt nach Aufarbeitung 5,60 g der obigen Verbindung 64.
d) 3,3-(2,2-Dimethyltrimethylendioxy)-5α-hydroxy-11β-[4-(3-pyridyl)-phenyl]-14β-estra-9,15-dien-17-on (65)
Aus 5,60 g der unter c) dargestellten Verbindung 64, 600 ml Essigester, 40 ml Triethylamin und 250 g Kieselgel werden nach Chromatographie an 550 g Kieselgel, wie unter Beispiel 2d) beschrieben, 3,2 g der obigen Verbindung 65 erhalten.
e) 3,3-(2,2-Dimethyltrimethylendioxy)-5α-hydroxy-11β-[4-(3-pyridyl)-phenyl]-14β-estr-9-en-17-on (66)
Eine Lösung von 3,39 g der unter d) hergestellten Verbindung 65 in 155 ml Ethanol wird mit 0,68 g Palladium/Calciumcarbonat (5% Pd) hydriert. Filtration über Celite und Entfernen des Lösungsmittels im Vakuum liefert nach Chromatographie mit Hexan/Essigester an Aluminiumoxid 2,62 g der obigen Verbindung 66.
f) 3,3-(2,2-Dimethyltrimethylendioxy)-17-[3-(tetrahydropyran-2-yl-oxy)-1-propin -yl]-11β-[4-(3-pyridyl)-phenyl]-14β-estr-9-en-5α,17α-diol (67)
Aus 6,95 g 3-(Tetrahydropyran-2-yloxy)-1-propin in 245 ml absolutem Tetrahydrofuran, 31 ml einer 1,6M Lösung von n-Butyllithium (Hexan) und 2,62 g der unter e) hergestellten Verbindung 66 in 53 ml Tetrahydrofuran werden, wie unter Beispiel 9c) beschrieben, nach Säulenchromatographie 2,3 g der obigen Verbindung 67 erhalten.
g) 3,3-(2,2-Dimethyltrimethylendioxy)-17-[3-(tetrahydropyran-2-yl-oxy)-prop-(Z)-1-enyl]-11β-[4-(3-pyridyl)-phenyl]-14β-estr-9-en-5α,17α-diol (68)
900 mg der unter f) hergestellten Verbindung 67 werden in 10 ml Ethanol gelöst und mit 0,9 ml Pyridin und 90 mg Palladium/Bariumsulfat (10% Pd) versetzt. Anschließend wird bei Normaldruck mit Wasserstoff hydriert. Nach Aufnahme eines Equivalents Wasserstoff wird der Katalysator durch Filtration über Celite abgetrennt, das Filtrat eingeengt und der Rückstand an Aluminiumoxid mit einem Gemisch aus Essigester/Hexan chromatographiert. Es werden 700 mg der obigen Verbindung 68 isoliert.
h) Nach der allgemeinen Vorschrift 2 wird die Titelverbindung 69 erhalten (Tabelle 2).

**Darstellung des Arylbromids**

4-(3-Pyridyl)-phenol

26,8 g 4-Bromphenol werden unter Schutzgas in 1000 ml Dioxan vorgelegt, mit 90 g 3-Tributylstannylpyridin [Darstellung nach Lit.: Org. Magn. Resonance, 7 (1975),610] und 11.1 g Bis-(triphenylphosphin)-palladium(II)chlorid versetzt und 15 Stunden unter Rückfluß erhitzt. Nach Einengen am Vakuum und Reinigung an Kieselgel werden 15,92 g obiger Verbindung isoliert.

4-(3-Pyridyl)-1-(trifluormethylsulfonyloxy)-benzol

15,92 g obiger Verbindung und 55,0 g 4-Dimethylaminopyridin werden unter Argon bei -78 °C in 900 ml Methylenchlorid vorgelegt und mit 19,8 ml Trifluormethansulfonsäureanhydrid gelöst in 300 ml absolutem Methylenchlorid tropfenweise versetzt. Nach 30 Minuten wird die Reaktionsmischung auf gesättigte Natriumhydrogencarbonatlösung gegossen. Nach weiteren 30 Minuten wird mit Methylenchlorid extrahiert, die organischen Phasen mit Kochsalzlosung gewaschen, über Natriumsulfat getrocknet und im Vakuum eingeengt. Nach Chromatographie an Kieselgel werden 26,0 g obiger Verbindung isoliert.

4-(3-Pyridyl)-1-tri-n-butylstannyl-benzol

Aus 26,0 g obiger Verbindung in 1300 ml Dioxan, 127,8 ml Hexabutyldizinn, 10,72 g Lithiumchlorid und 3,84 g Tetrakis-(triphenylphosphin)-palladium werden, wie unter Beispiel 5a) beschrieben, nach säulenchromatographischer Reinigung an Aluminiumoxid 21,4 g obiger Verbindung erhalten.

1-Brom-4-(3-pyridyl)-benzol

21,4 g obiger Verbindung in 220 ml Tetrachlorkohlenstoff werden bei -40 °C unter Argon tropfenweise mit 2,44 ml Brom unter heftigem Rühren versetzt. Man läßt auf Raumtemperatur kommen, wäscht mit

Kochsalzlösung, trocknet über Natriumsulfat und engt im Vakuum ein. Nach Chromatographie an Aluminiumoxid werden 10,2 g obiger Verbindung isoliert.

**BEISPIEL 15**

11β-[4-(3-Pyridyl)-phenyl]-17α-hydroxy-17-(3-hydroxypropyl)-14β-estra-4,9-dien-3-on (71) (Tabelle 2)

a) 3,3-(2,2-Dimethyltrimethylendioxy)-17-[3-(tetrahydropyran-2-yl-oxy)-propyl)-11β-[4-(3-pyridyl)-phenyl]-14β-estr-9-en-5α,17α-diol (70)
Aus 1,4 g der unter Beispiel 14f) dargestellten Verbindung 67 in 57 ml Ethanol und 140 mg Palladium/Kohle (10% Pd) werden nach Druckhydrierung (60 bar, Raumtemperatur) und üblicher Aufarbeitung 632 mg der obigen Verbindung 70 erhalten.
b) Nach der allgemeinen Vorschrift 2 wird die Titelverbindung 71 erhalten (Tabelle 2).

**BEISPIEL 16**

11β-[4-(3-Pyridyl)-phenyl]-14β-estra-4,9-dien-(17α,1'-spiro-17,2')-tetrahydrofuran-3-on (72) (Tabelle 2)

Aus 175 mg der unter Beispiel 15 dargestellten Verbindung 71 in 10 ml Methylenchlorid, 0,5 ml Triethylamin und 206 mg p-Toluolsulfonsäurechlorid werden, wie unter Beispiel 10 beschrieben, 135 mg der Titelverbindung 72 erhalten.
$[\alpha]_D^{22} = 201{,}0°$ ($CHCl_3$; C = 0,505)

Tabelle 1

| Beispiel | Ansatz | | | Reaktionsparameter | | Ausbeute | |
|---|---|---|---|---|---|---|---|
| | x[g] | y[ml] | z[ml] | t[°C] | a[min] | b[g] | $[\alpha]_D^{20}$ ($CHCl_3$) |
| 5 | 0.48 | 30 | 1.2 | 50 | 30 | 0.26 | 125.4(c = 1.87) |
| | Steroid 5d (31) | | | | | Steroid Bsp.5 (32) | |
| 6 | 0.55 | 30 | 1.4 | 45 | 60 | 0.26 | 106.0(c = 0.52) |
| | Steroid 6b (33) | | | | | Steroid Bsp.6 (34) | |
| 7 | 0.76 | 40 | 1.9 | 50 | 120 | 0.34 | 157.4(c = 0.51) |
| | Steroid 7b (35) | | | | | Steroid Bsp.7 (36) | |
| 8 | 0.72 | 40 | 1.9 | 50 | 30 | 0.33 | 161.5(c = 0.52) |
| | Steroid 8b (37) | | | | | Steroid Bsp.8 (38) | |
| 9 | 0.98 | 50 | 2.5 | 25 | 240 | 0.67 | 134.5(c = 0.52) |
| | Steroid 9f (50) | | | | | Steroid Bsp.9 (51) | |

Tabelle 2

| Beispiel | Ansatz | | Reaktionsparameter | | Ausbeute | |
|---|---|---|---|---|---|---|
| | x[g] | y[ml] | t[°C] | z[min] | a[g] | $[\alpha]_D^{22}$ ($CHCl_3$) |
| 14 | 0.70 | 9 | 50 | 60 | 0.33 | 253.5(c = 0.52) |
| | Steroid 14h(68) | | | | Steroid Bsp.14 (69) | |
| 15 | 0.63 | 8 | 50 | 60 | 0.35 | 222.6(c = 0.52) |
| | Steroid 15b(70) | | | | Steroid Bsp.15 (71) | |

## Patentansprüche

**1.** 11β-Phenyl-14βH-steroide der allgemeinen Formel I

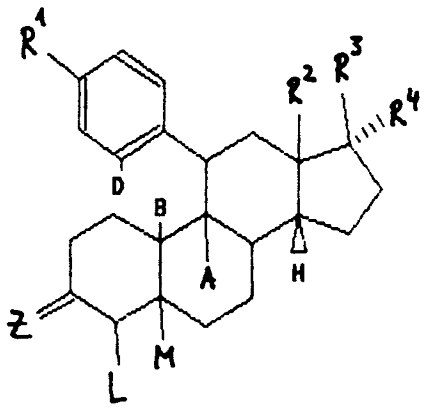

(I),

worin
Z für ein Sauerstoffatom oder die Hydroxyiminogruppierung N~OH sowie L und M entweder gemeinsam für eine zweite Bindung oder L für ein Wasserstoffatom und M für eine α-ständige Hydroxygruppe stehen und entweder A und B zusammen eine zweite Bindung und D ein Wasserstoffatom bedeuten wobei

$R^1$ entweder

a) für einen Heteroarylrest der Formel Ia

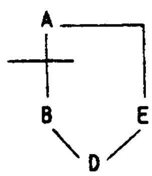

(Ia),

wobei A = N, O oder S und
B-D-E die Elementenfolge
C-C-C, N-C-C oder C-N-C
bedeuten oder

b) für einen Heteroarylrest der Formel Ib

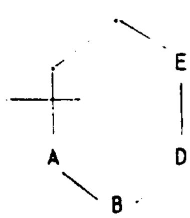

(Ib),

wobei A = N und
B-D-E die Elementenfolge
C-C-C, N-C-C, C-N-C oder C-C-N
bedeuten oder

c) für einen Cycloalkyl-, Cycloalkenyl- oder Arylrest Ic stehen oder aber A ein Wasserstoffatom und B und D zusammen eine das C-10-Atom des Steroidgerüstes und das ortho-Kohlenstoffatom des 11β-Phenylringes überbrückende Methylengruppe bedeuten, wobei dann Z zusätzlich für 2 Wasser-

stoffatome steht und

$R^1$ entweder die vorstehend in diesem Anspruch unter a), b) oder c) genannte Bedeutung hat oder

d) für einen geradkettigen, verzweigten, gesättigten oder ungesättigten Kohlenwasserstoffrest mit bis zu 10 Kohlenstoffatomen oder

e) für

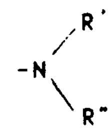

mit R′ und R″ in der Bedeutung von Wasserstoff oder Alkyl mit 1 bis 4 Kohlenstoffatomen oder R′ und R″ unter Einschluß von N in der Bedeutung eines gesättigten 5- oder 6gliedrigen Ringes, wobei im Ring außer N noch ein weiteres Heteroatom wie O, N, S enthalten sein kann, sowie die entsprechenden tertiären N-Oxide und die Säureadditionssalze oder

f) für OR‴ mit R‴ = H, $C_1$-$C_8$-Alkyl oder

g) für $SR^{IV}$ mit $R^{IV}$ in der Bedeutung von R‴ oder

h) für einen geradkettigen, verzweigten, gesättigten oder ungesättigten Kohlenwasserstoffrest mit bis zu 10 Kohlenstoffatomen, der durch eine oder mehrere Oxo-, Hydroxyimino-, $C_1$-$C_{10}$-Acyloxy- oder $OR^V$-Gruppe(n) mit $R^V$ in der Bedeutung eines Wasserstoffatoms oder eines $C_1$-$C_8$-Alkylrestes substituiert ist,

steht und wobei gegebenenfalls der Heteroarylrest der Formel Ia durch einen oder mehrere Halogenreste und/oder einen oder mehrere Alkylreste mit 1 bis 3 Kohlenstoffatomen und der Cycloalkyl-, Cycloalkenyl- oder Arylrest Ic durch eine oder mehrere Halogen-, gegebenenfalls geschützte Hydroxy-, Alkoxy-, gegebenenfalls in Form des Sulfoxids oder Sulfons und/oder des N-Oxids oxidierte Alkylthio- und/oder Dialkylaminoreste substituiert sind,

$R_2$ für einen Methyl- oder Ethylrest,

$R_3/R_4$ für $-(CH_2)_o-C{\equiv}CY/OR_5$ $-CH=CH-(CH_2)_kCH_2R_7/-OR_5$

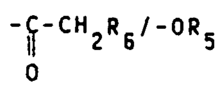 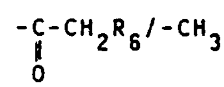

$-(CH_2)_oCH_2R_7/-OR_5$

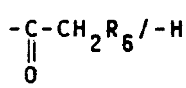

sowie, wenn A ein Wasserstoffatom und B und D zusammen einen das C-10-Atom des Steroidgerüstes und das ortho-C-Atom des 11β-Phenylringes überbrückende Methylengruppe bedeuten,

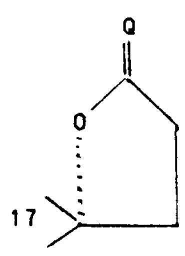

oder

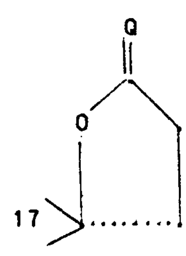

mit $R_5$ in der Bedeutung eines Wasserstoffatoms oder Acylrestes mit 1 bis 4 Kohlenstoffatomen und Y in der Bedeutung eines Wasserstoff-, Chlor-, Jod- oder Bromatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,

$R_6$ für ein Wasserstoffatomen, eine Hydroxygruppe, eine Alkyl-, O-Alkyl- oder O-Acylgruppe mit jeweils 1 bis 4 Kohlenstoffatomen,

O für die Ziffern 0, 1, 2 oder 3,
$R_7$ für einen Hydroxy- oder Cyanidrest, eine O-Alkyl- oder O-Acylgruppe mit jeweils 1 bis 4 Kohlenstoffatomen und
k für die Ziffern 0, 1 oder 2 und
Q für ein Sauerstoffatom oder zwei Wasserstoffatome stehen.

**2.** 13-Alkylgonane nach Anspruch 1, worin der Heteroarylrest der Formel Ia der 3-Thienyl-, 3-Furyl- oder 3-Pyrrolylrest ist.

**3.** 13-Alkylgonane nach Anspruch 1, worin der Heteroarylrest der Formel Ib der 3- oder 4-Pyridyl-, der 5-Pyrimidin-, 4-Pyridazin- oder Pyrazinrest ist.

**4.** 13-Alkylgonane nach Anspruch 1, worin der Cycloalkyl-, Cycloalkenyl-oder Arylrest Ic der Cyclohexyl-, ein Cyclohexenyl- oder der Phenylrest ist.

**5.** 13-Alkylgonane nach Anspruch 1, worin der Alkenylrest Id bis zu drei Doppelbindungen aufweist.

**6.** 13-Alkylgonane nach Anspruch 1, worin der Heteroarylrest der Formel Ia durch ein Chlor- oder Bromatom substituiert ist.

**7.** 13-Alkylgonane nach Anspruch 1, worin der Heteroarylrest der Formel Ia durch einen Alkylrest mit 1 bis 3 Kohlenstoffatomen substituiert ist.

**8.** 13-Alkylgonane nach Anspruch 1, worin der Cycloalkyl-, Cycloalkenyl-oder Arylrest Ic durch ein oder zwei Chlor- und/oder Bromatom(e) substituiert ist.

**9.** 13-Alkylgonane nach Anspruch 1, worin der Cycloalkyl-, Cycloalkenyl-oder Arylrest durch ein oder zwei, gegebenenfalls geschützte Hydroxy-und/oder Alkoxyreste mit 1 bis 8 Kohlenstoffatomen substituiert ist.

**10.** Verfahren zur Herstellung von Verbindungen der allgemeinen Formel I

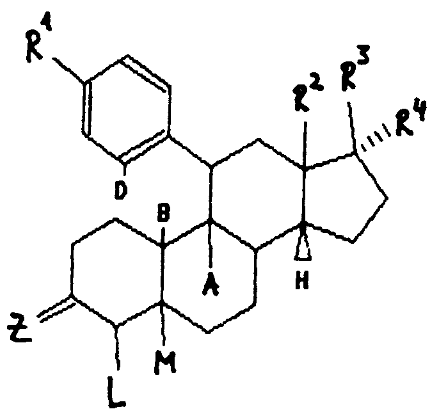

(I),

worin A, B, D, L, M, Z, $R^1$, $R^2$, $R^3$ und $R^4$ die in Anspruch 1 genannte Bedeutung haben, dadurch gekennzeichnet, daß eine Verbindung der allgemeinen Formel II

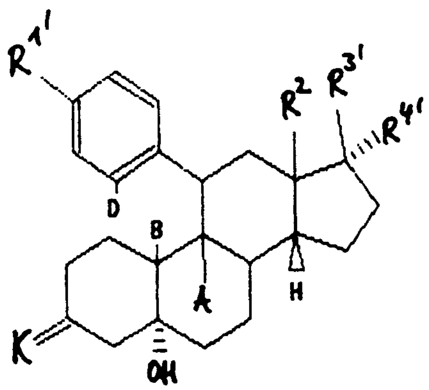

(II),

worin A, B, D und $R^2$ die in Formel I angegebene Bedeutung haben,

K eine in Form des Ketals oder Thioketals blockierte Ketogruppe bedeutet,

$R^{1'}$ entweder die bei $R^1$ unter a) bis g) angegebene Bedeutung hat, oder

einen geradkettigen oder verzweigten, gesättigten oder ungesättigten Alkylrest mit 1 bis 10 Kohlenstoffatomen, der die Gruppierung

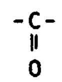

in der geschützten Form

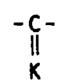

mit K in oben angegebener Bedeutung

oder K in der Bedeutung eines Wasserstoffatoms und einer gegebenenfalls geschützten Hydroxygruppe enthält, sowie

$R^{3'}$ und $R^{4'}$

die gleiche Bedeutung haben wie $R^3$ und $R^4$ in Formel I, wobei vorhandene Hydroxy- und/oder Acyl- und/oder terminale Alkingruppen gegebenenfalls geschützt sind,

der Einwirkung eines Dehydratationsmittels, das auch zur Freisetzung der 3-Oxogruppe und in $R^{1'}$ vorhandener geschützter Ketogruppen befähigt ist, gegebenenfalls unter Ausbildung der 4(5)-Doppelbindung unterwirft, das so erhaltene Produkt gegebenenfalls von weiteren Schutzgruppen befreit und gewünschtenfalls mit Hydroxylamin-hydrochlorid zum Produkt der allgemeinen Formel I mit Z in der Bedeutung der Hydroxyiminogruppierung N~OH umsetzt.

**11.** Pharmazeutische Präparate, gekennzeichnet durch einen Gehalt an Verbindungen gemäß den Ansprüchen 1 bis 9.

**12.** Verwendung von Verbindungen gemäß Anspruch 1 zur Herstellung von Arzneimitteln.

## Claims

**1.** 11$\beta$-phenyl-14$\beta$H-steroids of the general formula I

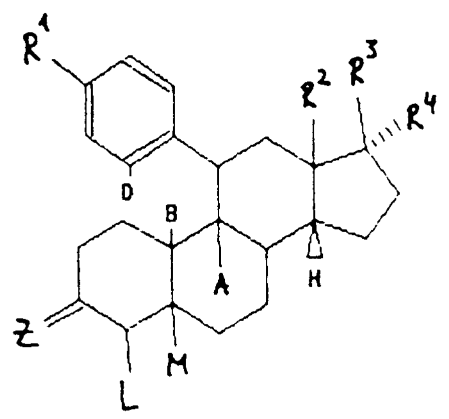

(I),

wherein

Z is an oxygen atom or the hydroxyimino grouping N~OH, and either L and M together form a second bond or L is a hydrogen atom and M is a hydroxy group in the $\alpha$-configuration, and either A and B together form a second bond and D is a hydrogen atom, in which case

$R^1$ is either

a) a heteroaryl radical of formula Ia

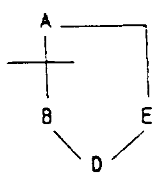

(Ia),

wherein A = N, O or S and

B-D-E is the element sequence

C-C-C, N-C-C or C-N-C,

or

b) a heteroaryl radical of formula Ib

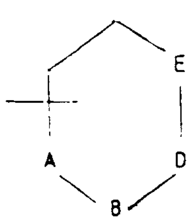

(Ib),

wherein A = N and

B-D-E is the element sequence

C-C-C, N-C-C, C-N-C or C-C-N,

or

c) a cycloalkyl, cycloalkenyl or aryl radical Ic

or A is a hydrogen atom and B and D together are a methylene group bridging the C-10 atom of the steroid ring system and the ortho-carbon atom of the 11$\beta$-phenyl ring, in which case Z additionally represents 2 hydrogen atoms and

$R^1$ either has the meaning give above in this claim under a), b) or c) or is

d) a straight-chained, branched, saturated or unsaturated hydrocarbon radical having up to 10 carbon atoms, or

e)

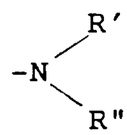

wherein R' and R" are hydrogen or alkyl having from 1 to 4 carbon atoms, or R' and R", with the inclusion of N, form a saturated 5- or 6-membered ring, it being possible for the ring to contain, in addition to N, also a further hetero atom, such as O, N or S, and the corresponding tertiary N-oxides and the acid addition salts, or

f) OR''' wherein R''' = H or $C_1$-$C_8$ alkyl, or

g) $SR^{IV}$ wherein $R^{IV}$ has the meaning of R''', or

h) a straight-chained, branched, saturated or unsaturated hydrocarbon radical having up to 10 carbon atoms that is substituted by one or more oxo, hydroxyimino, $C_1$-$C_{10}$ acyloxy or $OR^V$ group(s) wherein $R^V$ is a hydrogen atom or a $C_1$-$C_8$ alkyl radical,

and wherein, if desired, the heteroaryl radical of formula Ia is substituted by one or more halogen radicals and/or one or more alkyl radicals having from 1 to 3 carbon atoms, and the cycloalkyl, cycloalkenyl or aryl radical Ic is substituted by one or more halogen radicals, optionally protected hydroxy or alkoxy radicals, or by alkylthio and/or dialkylamino radicals optionally oxidised in the form of the sulfoxide or sulfone and/or of the N-oxide,

$R_2$ is a methyl or ethyl radical,

$R_3/R_4$ are $-(CH_2)_o-C\equiv CY/OR_5$ $\quad -CH=CH-(CH_2)_kCH_2R_7/-OR_5$

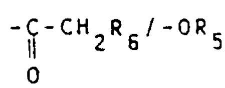

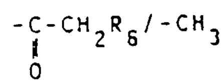

$-(CH_2)_oCH_2R_7/-OR_5$

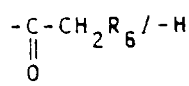

and, when A is a hydrogen atom and B and D together are a methylene group bridging the C-10 atom of the steroid ring system and the ortho-C atom of the 11$\beta$-phenyl ring,

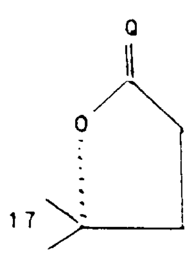

or

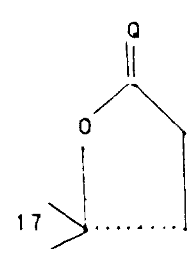

wherein $R_5$ is a hydrogen atom or an acyl radical having from 1 to 4 carbon atoms

and Y is a hydrogen, chlorine, iodine or bromine atom, or an alkyl, hydroxyalkyl, alkoxyalkyl or acyloxyalkyl group having from 1 to 4 carbon atoms in each alkyl or acyl radical,

$R_6$ is a hydrogen atom or a hydroxy group, or an alkyl, O-alkyl or O-acyl group having from 1 to 4 carbon atoms,

0 is the number 0, 1, 2 or 3,

$R_7$ is a hydroxy or cyanide radical, or an 0-alkyl or 0-acyl group having from 1 to 4 carbon atoms, and
k is the number 0, 1 or 2, and
Q is an oxygen atom or two hydrogen atoms.

**2.** 13-Alkylgonanes according to claim 1, wherein the heteroaryl radical of formula Ia is the 3-thienyl, 3-furyl or 3-pyrrolyl radical.

**3.** 13-Alkylgonanes according to claim 1, wherein the heteroaryl radical of formula Ib is the 3- or 4-pyridyl, 5-pyrimidine, 4-pyridazine or pyrazine radical.

**4.** 13-Alkylgonanes according to claim 1, wherein the cycloalkyl, cycloalkenyl or aryl radical Ic is the cyclohexyl radical, a cyclohexenyl radical or the phenyl radical.

**5.** 13-Alkylgonanes according to claim 1, wherein the alkenyl radical Id has up to three double bonds.

**6.** 13-Alkylgonanes according to claim 1, wherein the heteroaryl radical of formula Ia is substituted by a chlorine or bromine atom.

**7.** 13-Alkylgonanes according to claim 1, wherein the heteroaryl radical of formula Ia is substituted by an alkyl radical having from 1 to 3 carbon atoms.

**8.** 13-Alkylgonanes according to claim 1, wherein the cycloalkyl, cycloalkenyl or aryl radical Ic is substituted by one or two chlorine and/or bromine atom(s).

**9.** 13-Alkylgonanes according to claim 1, wherein the cycloalkyl, cycloalkenyl or aryl radical is substituted by one or two optionally protected hydroxy and/or alkoxy radicals having from 1 to 8 carbon atoms.

**10.** Process for the preparation of compounds of the general formula I

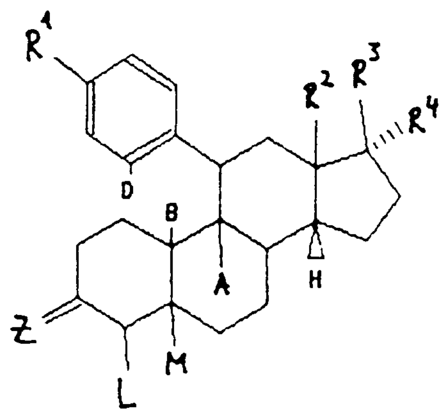

(I),

wherein A, B, D, L, M, Z, $R^1$, $R^2$, $R^3$ and $R^4$ have the meaning given in claim 1, characterised in that a compound of the general formula II

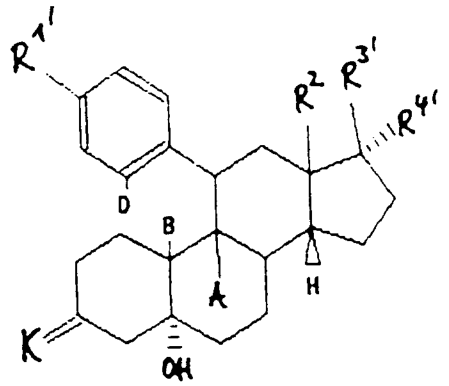

(II).

wherein A, B, D and $R^2$ have the meaning given in formula I,

K is a keto group blocked in the form of the ketal or thioketal,

$R^{1'}$ either has the meaning given for $R^1$ in a) to g) or

is a straight-chained or branched, saturated or unsaturated alkyl radical having from 1 to 10 carbon atoms which contains the grouping

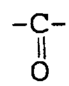

in the protected form

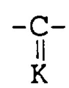

wherein K has the meaning given above or

K has the meaning of a hydrogen atom and an optionally protected hydroxy group, and

$R^{3'}$ and $R^{4'}$ have the same meaning as $R^3$ and $R^4$ in formula I, any hydroxy and/or acyl and/or terminal alkyne groups present being optionally protected,

is subjected to the action of a dehydrating agent that is also capable of freeing the 3-oxo group and protected keto groups present in $R^{1'}$, if desired with the formation of the 4(5)-double bond, the product thus obtained is, where appropriate, freed of further protecting groups and, if desired, is reacted with hydroxylamine hydrochloride to form the product of the general formula I wherein Z is the hydroxyimino grouping N~OH.

**11.** Pharmaceutical preparations, characterised in that they contain compounds according to claims 1 to 9.

**12.** Use of compounds according to claim 1 for the preparation of medicaments.

## Revendications

**1.** 11β-phényl-14βH-stéroïdes de formule générale I

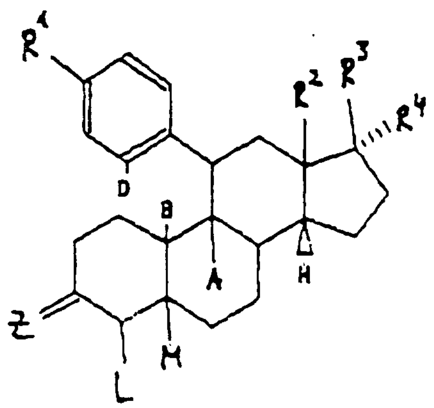

(I).

dans laquelle
Z représente un atome d'oxygène ou le groupement hydzoxyimino N~OH et
L et M soit représentent ensemble une deuxième liaison, soit L représente un atome d'hydrogène et M représente un groupe hydroxy en position α et
soit A et B représentent ensemble une deuxième liaison et D un atome d'hydrogène,
R1 représentant soit
a) un reste hétéroaryle de formule Ia

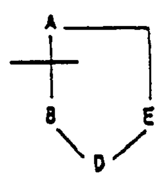

(Ia),

dans laquelle A signifie N, O ou S et B-D-E signifie la suite des éléments C-C-C, N-C-C ou C-N-C soit
b) un reste hétéroaryle de formule I b

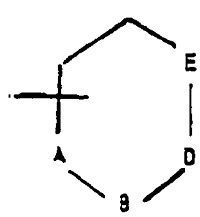

(Ib),

dans laquelle A signifie N et
B-D-E signifie la suite des éléments C-C-C, N-C-C, C-N-C ou C-C-N soit encore
c) un reste cycloalkyle, cycloalcényle ou bien aryle Ic
soit A signifie un atome hydrogène et B et D signifient, ensemble, un groupe méthylène reliant par un pont l'atoms C-10 du squelette de stéroïde et l'atome de carbone en position ortho du cycle phényle, lui même en position 11β,
Z représentant alors, de plus, 2 atomes d'hydrogène et
$R^1$ ayant soit la signification mentionnée précédemment dans cette revendication sous le a), le b) ou c),
soit représentant

d) un reste hydrocarboné à chaîne droite ou ramifiée, saturé ou non saturé, ayant jusqu'à 10 atomes de carbone ou bien

e) avec R' et R'' signifiant un

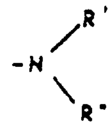

hydrogène ou un alkyle ayant de 1 à 4 atomes de carbone ou bien R' et R'' représentant un cycle saturé à 5 ou 6 chaînons avec inclusion de N, un autre hétéroatome tel que O, N, S pouvant être contenu, outre N, dans le cycle, ainsi que le N-oxyde tertiaire correspondant et le sel d'addition d'acide ou bien

f) OR''' avec R''' étant un H, un alkyle en $C_1$-$C_8$ ou bien

g) $SR^{IV}$ avec $R^{IV}$ ayant la signification de R''' ou bien

h) un reste hydrocarboné à chaîne droite ou ramifiée, saturé ou non saturé, ayant jusqu'à 10 atomes de carbone qui est substitué par un ou plusieurs groupes oxo, hydroxyimino, alcoxy en $C_1$-$C_{10}$ ou bien $OR^V$ avec $R^V$ signifiant un atome hydrogène ou un reste alkyle en $C_1$-$C_8$,

et éventuellement le reste hétéroaryle de formule Ia étant substitué par un ou plusieurs restes halogène et/ou un ou plusieurs restes alkyle ayant de 1 à 3 atomes de carbone

et les restes cycloalkyle, cycloalcényle ou aryle Ic étant substitués par un ou plusieurs restes halogène, hydroxy eventuellement protégés, alcoxy, alkylthio et/ou dialkylamino éventuellement oxydés sous la forme du sulfoxyde ou de la sulfone et/ou du N-oxyde,

$R^2$ représente un reste méthyle ou éthyle, $R_3$/$R_4$ représentent

$-(CH_2)_o-C\equiv CY/OR_5$ $-CH=CH-(CH_2)_kCH_2R_7/-OR_5$

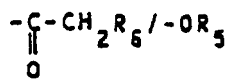 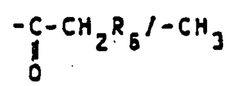

$-(CH_2)_oCH_2R_7/-OR_5$

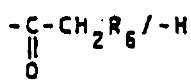

ainsi que, lorsque A signifie un atome d'hydrogène et B et D signifient, ensemble, un groupe méthylène reliant par un pont l'atome C-10 du squelette de stéroïde et l'atome de carbone en position ortho du cycle phényle, lui même en position 11$\beta$,

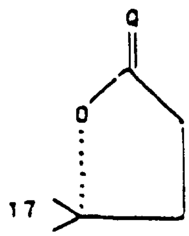 ou 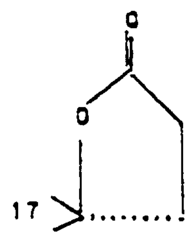

avec

$R^5$ signifiant un atome hydrogène ou un reste acyle ayant de 1 à 4 atomes de carbone et

Y signifiant un atome hydrogène, de chlore, d'iode ou de brome, un groupe alkyle, hydroxyalkyle, alcoxyalkyle ou acyloxyalkyle avec à chaque fois de 1 à 4 atomes de carbone dans le reste alkyle ou bien acyle,

$R_6$ représentant un atome hydrogène, un groupe hydroxy, un groupe alkyle, O-alkyle ou O-acyle avec

à chaque fois de 1 à 4 atomes de carbone, o représentant les chiffres 0, 1, 2 ou 3,
$R_7$ représentant un reste hydroxy ou cyanure, un groupe O-alkyle ou O-acyle avec à chaque fois de 1 à 4 atomes de carbone et
k représentant les chiffres 0, 1 ou 2 et
Q représentant un atome oxygène ou deux atomes d'hydrogène.

**2.** 13-alkylgonanes selon la revendication 1, dans lesquels le reste hétéroaryle de formule Ia est le reste 3-thiényle, 3-furyle ou 3-pyrrolyle.

**3.** 13-alkylgonanes selon la revendication 1, dans lesquels le reste hétéroaryle de formule Ib est le reste 3-ou 4- pyridyle, 5-pyrimidine, 4-pyridazine ou pyrazine.

**4.** 13-alkylgonanes selon la revendication 1, dans lesquels le reste cycloalkyle, cycloalcényle ou aryle Ic est le reste cyclohexyle, un reste cyclohexényle ou le reste phényle.

**5.** 13-alkylgonanes selon la revendication 1, dans lesquels le reste alcényle Id possède jusqu'à trois doubles liaisons.

**6.** 13-alkylgonanes selon la revendication 1, dans lesquels le reste hétéroaryle de formule Ia est substitué par un atome de chlore ou de brome.

**7.** 13-alkylgonanes selon la revendication 1, dans lesquels le reste hétéroaryle de formule Ia est substitué par un reste alkyle ayant de 1 à 3 atomes de carbone.

**8.** 13-alkylgonanes selon la revendication 1, dans lesquels le reste cycloalkyle, cycloalcényle ou aryle Ic est substitué par un ou deux atomes de chlore et/ou de brome.

**9.** 13-alkylgonanes selon la revendication 1, dans lesquels le reste cycloalkyle, cycloalcényle ou aryle est substitué par un ou deux restes hydroxy éventuellement protégés et/ou alcoxy ayant de 1 à 8 atomes de carbone.

**10.** Procédé de préparation des composés de formule générale I

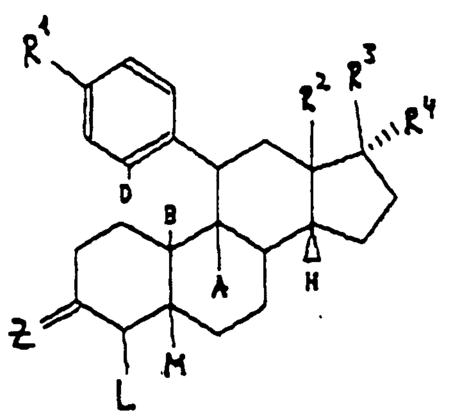

(I),

dans laquelle
A, B, D, L, M, Z, R1, R2, R3 et R4 ont la
signification mentionnée dans la revendication 1, caractérisé en ce qu'un composé de formule générale II

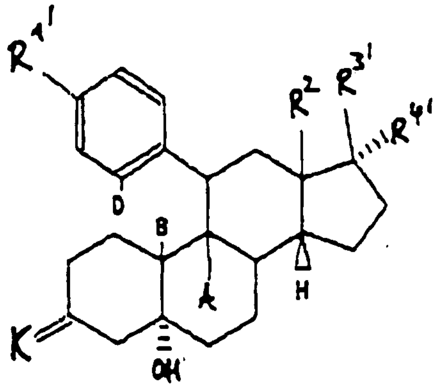

(II).

dans laquelle

A, B, D et $R^2$ ont la signification donnée dans la formule I,

K signifie un groupe céto bloqué sous la forme du cétal ou du thiocétal,

$R^{1'}$ a la signification donnée pour R1 dans a) à g), ou alors signifie un reste alkyle à chaîne droite ou ramifiée, saturé ou insaturé, ayant de 1 à 10 atomes de carbone, qui contient le groupement

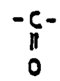

sous la forme protégée

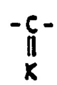

avec K ayant la signification donnée ci-dessus ou bien signifiant un atome d'hydrogène ou un groupe hydroxy éventuellement protégé, ainsi que

$R^{3'}$ et $R^{4'}$ ont la même signification que pour $R^3$ et $R^4$ dans la formule I, les groupes hydroxy et/ou acyle et/ou alcyne terminaux présents étant éventuellement protégés,

est soumis à l'action d'un agent de déshydratation, qui est susceptible aussi de libérer le groupe oxo en position 3 ainsi que les groupes céto protégés présents dans $R^{1'}$, en formant éventuellement la double liaison en position 4(5), le produit ainsi obtenu est éventuellement débarrassé des autres groupements protecteurs et, si on le souhaite, est mis en réaction avec le chlorhydrate de l'hydroxylamine pour donner le produit de formule générale I avec Z signifiant le groupement hydroxyimino N~OH.

**11.** Préparation pharmaceutique, caractérisée en ce qu'elle contient un composé selon les revendications 1 à 9.

**12.** Utilisation des composés selon la revendication 1 pour la préparation de médicaments.